## Europäisches Patentamt
## European Patent Office
## Office européen des brevets

(19)

(11) Publication number: **0 001 114**
**B1**

(12) # EUROPEAN PATENT SPECIFICATION

(45) Date of publication of patent specification: **07.07.82**

(51) Int. Cl.³: **A 61 M 5/14**

(21) Application number: **78100834.7**

(22) Date of filing: **06.09.78**

(54) Intravenous solution flow regulator.

(30) Priority: **07.09.77 US 831129**

(43) Date of publication of application:
**21.03.79 Bulletin 79/6**

(45) Publication of the grant of the patent:
**07.07.82 Bulletin 82/27**

(84) Designated Contracting States:
**BE DE FR GB SE**

(56) References cited:
**DE - A - 2 729 642**
**DE - B - 1 766 980**
**FR - A - 1 171 578**
**FR - A - 1 179 425**
**FR - A - 1 358 390**
**FR - A - 1 546 081**
**FR - A - 2 112 208**
**FR - A - 2 253 544**
**FR - A - 2 341 841**
**GB - A - 990 583**
**US - A - 2 562 445**

(73) Proprietor: **Becker, Karl Edmund, Dr.**
**275 S. Pershing Drive**
**Wichita Kansas 67218 (US)**

(72) Inventor: **Becker, Karl Edmund, Dr.**
**275 S. Pershing Drive**
**Wichita Kansas 67218 (US)**

(74) Representative: **Seidler, Bernhard et al,**
**Lorenz & Seidler Widenmayerstrasse 23**
**D-8000 München 22 (DE)**

(56) References cited:
**US - A - 3 738 361**
**US - A - 3 989 043**
**US - A - 3 999 542**
**US - A - 4 013 072**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Intravenous solution flow regulator

This invention relates to an automatic flow regulator for delivering a fluid, such as a parenteral solution, from a fluid source to a patient, the flow regulator comprising: a body portion having an inlet for receiving fluid from said fluid source, an outlet for delivering fluid to said patient, and a flow path for delivering fluid from said inlet to said outlet; an elongated piercer on said body portion proximate said inlet and having a primary fluid inlet at the end remote from said body portion. More particularly this invention relates to a flow regulator which sustains fluid flow to the patient at a reduced rate after the level of fluid in the intravenous fluid (IV) container drops below a predetermined threshold, and to a flow regulator which provides accurate regulation of the total flow rate of the intravenous infusion. In one design, in addition to the above stated functions, total flow rate is adjustable and may be maintained very constant over the time of infusion, and complete filtration of the parenteral solution fluid is provided. In another design, the total flow rate is fixed at a maximum level, and minor adjustments to the flow rate are accomplished by changing the height of the IV container relative to the patient.

US—A—4,013,072 discloses apparatus for regulating the flow of parenteral fluid comprising a hollow body 44 having an inlet 46 and an outlet 48, and a hydrophilic filter element 51 which is disposed between the inlet and outlet and is capable of passing liquid therethrough while simultaneously blocking air. The patent does not disclose or suggest to use the filter element to set a predetermined maximum flow rate.

FR—A—1,546,081 discloses apparatus which is similar to that of US—A—4,013,072 except for the location of the filter.

FR—A—1,179,425 discloses a salt shaker comprising a hollow body 11 having an inlet 19 and an outlet 45 and a filter 49 located between the inlet and the outlet. That patent is directed to an entirely different field and does not disclose filter membranes and flow regulating plate means which cooperate during relative rotation to vary the rate of flow of solution by varying the effective area of the filter membrane.

In my German patent application, DE—A—2,729,642.0, filed June 30, 1977, I explained the desirability of maintaining the flow of parenteral solution for an extended period of time after the IV container becomes empty, and the problems which result when fluid flow ceases. Briefly stated, if the flow of parenteral solution ceases, even for a brief period of time, the intravenous infusion catheter often becomes clotted, necessitating an intravenous catheter or needle change, with the accompanying discomfort to the patient, incurred cost of materials, and loss of employee time.

In my aforementioned patent application, there is disclosed a mechanism which associates with an IV container, and which maintains fluid flow for a prolonged period of time even when the container is empty. This mechanism includes a reservoir chamber for receiving and storing parenteral solution from the IV container, and a valving arrangement for automatically decreasing the fluid flow rate to a lower secondary rate once the infusion bottle is empty and fluid level in the reservoir drops below a predetermined threshold level.

A main purpose of the present invention is to accomplish reduced fluid flow when the IV container becomes nearly empty, without the necessity for a special reservoir chamber and its associated valving.

Another main purpose of this present invention is to provide accurate flow rate regulation. Flow rate in most intravenous administration sets is regulated by roller clamps or other means of constricting the orifice of the IV infusing tubing to limit the flow of fluid. However, due to "creep" or "cold flow" of the plastic employed in the IV tubing, flow tends to decrease with time and often will decrease as much as 60% of the desired rate within one hour. Such inaccurate flow regulation necessitates frequent monitoring by hospital personnel to maintain the correct flow. This problem not only results in a waste of valuable medical personnel time but also increases the possibility that the intravenous infusion may be stopped prematurely. To solve this longstanding problem, recently many expensive electronic and magnetic monitoring and motorized pumping devices have become available. These devices, although solving to some extent the present problem, are expensive, cost much more than a simple infusion set, and require frequent nursing personnel visits to assure that the devices remain operational.

The present invention relates to a flow regulator having integral capabilities for prolonging the time of flow of the parenteral solution at a reduced rate, when the level of fluid in the container reaches a certain threshold level. The present invention provides the advantages of a maintained continuous fluid flow without the necessity for adding additional components such as reservoirs or the like to an existing intravenous infusion set.

The inventive flow regulator is adapted for direct mating with a standard IV container. The present invention provides apparatus having a primary, high flow rate fluid path, a secondary, low flow rate fluid path, and a regulator means to cut off the primary fluid flow path to both fluid and air flow when the fluid level in the IV bottle falls below a threshold level. In one embodiment, the flow regulating means or valving means is located external to the IV bottle when the piercer is in place. In another embodiment, the valving or regulating mechanism resides inside the IV container after the piercer has been inserted therein.

The flow regulating means can be embodied in different forms incorporating the teachings of the present invention. For example, the flow regulating valving means may be a hydrophilic membrane filter than prohibits the passage of air, or it may be in the form of a conventional flap or ball valve mechanism. The specific configuration of the flow regulator need only be so constructed so as to accomplish the stated purpose of enabling a rapid flow rate of parenteral solution until the level of solution in the IV container reaches a certain lower threshold level, whereupon the fluid flow is then restricted to a lower flow rate, thereby extending the period of time during which the last portion of parenteral solution in the IV container is infused to the patient.

The present invention provides apparatus which performs the flow regulating function and which also has an integral means for accurately controlling the fluid flow rate. One embodiment of the present invention provides accurate flow rate regulation by utilizing a tubular flow path, of selected length and inner diameter, so as to deliver a flow rate typical of conventional IV infusion sets when the IV container is located at an average height above the patient. In another embodiment of the present invention, accurate and constant primary and secondary flow rate regulation is provided by the use of membrane filters. As is well known, the flow through these filters is proportional to the pore size of the filter, the effective surface area of the filter, and the fluid pressure head as determined by the height of the infusion fluid level relative to the plane of the patient. These parameters may be chosen such that the present invention provides a conventional intravenous flow rate which will change, only one to two percent per hour of infusion. And, in fact, this change in flow rate is caused almost entirely by the decrease in the height of the intravenous infusion solution from the continued infusion.

The present invention provides apparatus employing several means for easily adjusting this flow rate, for example, by changing the effective area of the filter membrane, or by changing the height of the IV infusion container relative to the patient, or both. In any event, the flow resulting from the use of the present invention is much more constant and easier to regulate than the conventional IV infusion sets which use clamps or the like to regulate and control the flow by restricting the feed tubing inner diameter. In addition, the present invention also provides apparatus which is adapted to completely filter the intravenous fluid which is being infused to the patient. Accordingly, it may be seen that the principal objectives of the present invention are to extend the time duration of IV infusion to a patient when the IV container becomes nearly empty, to accurately regulate the rate of flow of the IV infusion fluid, and to perform both of these objects in a single integral device and to provide such device with means to provide complete filtration of the IV infusion fluid.

Therefore, it is a main object of the present invention to provide an intravenous flow regulator which automatically extends the time during which parenteral fluid flows to a patient when the infusion bottle becomes nearly empty.

It is another object of the present invention to provide an IV flow regulator with an integral valving mechanism which becomes operational upon the parenteral fluid reaching a predetermined threshold level.

It is still a further object of the present invention to provide an integral piercer and flow regulator which includes valving means adapted to reside within the IV container when in use.

It is still a further object of the present invention to provide a flow regulator for an IV container, which flow regulator includes valving means adapted to reside external to the IV container when in use.

A more specific object of the present invention is to provide an intravenous flow regulator having a primary flow path which is intended to deliver the parenteral solution to the patient when the IV container has an adequate fluid supply and having a mechanism for closing this primary path to air and fluid entry once the fluid level in the IV container reaches a predetermined threshold level, and employing a secondary reduced rate flow path which is solely responsible for the infusion to the patient after the threshold level has been reached.

A still further specific object of the present invention is to provide an intravenous flow regulator having a continuously active secondary flow path which becomes the sole source of infusion fluid when the primary flow path has been closed.

It is another main object of the present invention to provide an intravenous flow regulator which accurately regulates intravenous flow rate.

It is another object of the present invention to provide an intravenous flow regulator providing accurate and continuously maintained flow rate regulation and employing a filter membrane of selected effective surface area and pore size thereby providing a fixed flow rate commensurate with typical intravenous infusion flow rates.

It is still a further object of the present invention to provide an intravenous flow regulator having a primary filter membrane and including a means for adjusting the effective surface area of the membrane presented to the fluid, thereby providing accurate flow rate adjustment.

It is still a further object of the present invention to provide an automatic flow regulator employing a mechanism for limiting the flow to a predetermined fixed maximum when the IV container is relatively full and for automatically reducing the fluid flow to a predetermined lower flow rate when the IV container is almost empty.

It is another object of the present invention to provide an automatic intravenous flow regulator having primary high flow rate, and secondary low flow rate fluid paths, and having a primary membrane filter of selected pore size located therein with means to adjust the surface area of the membrane for principally determining the primary flow rate, and having a secondary filter membrane of selected pore size and surface area for determining secondary flow rate and having a mechanism for closing off the primary fluid path to the passage of fluid when the IV container becomes nearly empty, and utilizing the hydrophilic quality of the primary and secondary membranes to block the passage of air through these membranes when they are no longer in contact with fluid and utilizing also the filtration properties of the membranes to completely filter the IV fluid infused to the patient.

Still another object of the present invention is to provide an intravenous flow regulator having a primary membrane filter of selected pore size and effective surface area so as to provide an IV flow rate commensurate with typical IV infusions and to provide filtration of particles and bacteria, and having a secondary membrane filter of selected pore size and effective surface area to provide a low flow rate and to provide particle and bacteria filtration and having a means for stopping flow through the primary membrane when the level of fluid in the IV container reaches a predetermined threshold level.

It is yet another object of the present invention to provide a flow regulator utilizing integral tubular flow paths of chosen diameter and length to provide predetermined fluid flow rates in dependence upon the level of parenteral solution within the IV container.

Flow regulators designed to accomplish objects similar to those underlying the invention but using different means for this purpose have been disclosed in US—A—3,999,542 and US—A—3,738,361.

In order to accomplish these and other objects of the invention, the automatic flow regulator of the kind described first hereinbefore is characterized in accordance with the invention in that the piercer having a secondary fluid inlet positioned intermediate said primary fluid inlet and said body portion, and having at least one piercer flow path for delivering fluid entering said primary and secondary fluid inlets to said body portion inlet; whereby when said piercer is in said fluid source and is in communication with said fluid, said primary fluid inlet defines a threshold level whereabove fluid flows from said source to said outlet, through said primary and secondary fluid inlets, at a first flow rate; and valving means for preventing the flow of air from said primary fluid inlet to said outlet upon the level of said fluid in said source falling below said threshold level; whereby, after the level of fluid in said source falls below said threshold level, fluid flows from said source to said outlet, through only said secondary fluid inlet, at a second flow rate less than said first flow rate.

These and other objects of the present invention as well as many of the attendant advantages thereof, will become more readily apparent when reference is made to the following description, taken in conjunction with the accompanying drawings.

Figure 1 illustrates a first embodiment of the present invention inserted in an IV container;

Figure 2 is an alternate embodiment of the present invention;

Figure 3 is another embodiment of the present invention inserted in an IV container;

Figure 4A is a cross section of the embodiment of Figure 3, taken along site line 4A—4A;

Figure 4B is a cross section of the embodiment of Figure 3, taken along site line 4B—4B;

Figure 5 is a graph of a typical parenteral solution flow pattern when using the embodiment of the present invention illustrated in Figure 3;

Figure 6 is another embodiment of the present invention inserted in a plastic collapsible IV container;

Figure 7 is a cross section of the embodiment of Figure 6, taken along site line 7—7 in the plane of the drawing;

Figure 8 is a cross section of the embodiment of Figure 6, taken along site line 8—8;

Figure 9 is a cross section of the embodiment of Figure 6, taken along site line 9—9 in the plane of the drawing;

Figure 10 is a cross section of the embodiment of Figure 6, taken along site line 10—10;

Figure 11 is a side elevation of the sleeve portion of the embodiment shown in Figure 6;

Figure 12 illustrates another embodiment of the present invention inserted in an IV container;

Figure 13 is a cross section of the embodiment of Figure 12, taken along site line 13—13;

Figure 14 is a graph of the parenteral solution flow pattern utilizing the embodiment of the present invention illustrated in Figure 12;

Figure 15 illustrates another embodiment of the present invention;

Figure 16A is a cross section of the embodiment of Figure 15, taken along site line 16A—16A;

Figure 16B is a cross section of the embodiment of Figure 15, taken along site line 16B—16B;

Figure 17 is a cross section of the embodiment of Figure 15, taken along site line 17—17;

Figure 18 is a cross section of the embodiment of Figure 15, taken along site line 18—18;

Figure 19 is an alternate embodiment of the baffle, or flow regulator plate shown in Figure 17; and

Figure 20 is an alternate embodiment of the membrane carrier shown in Figure 18.

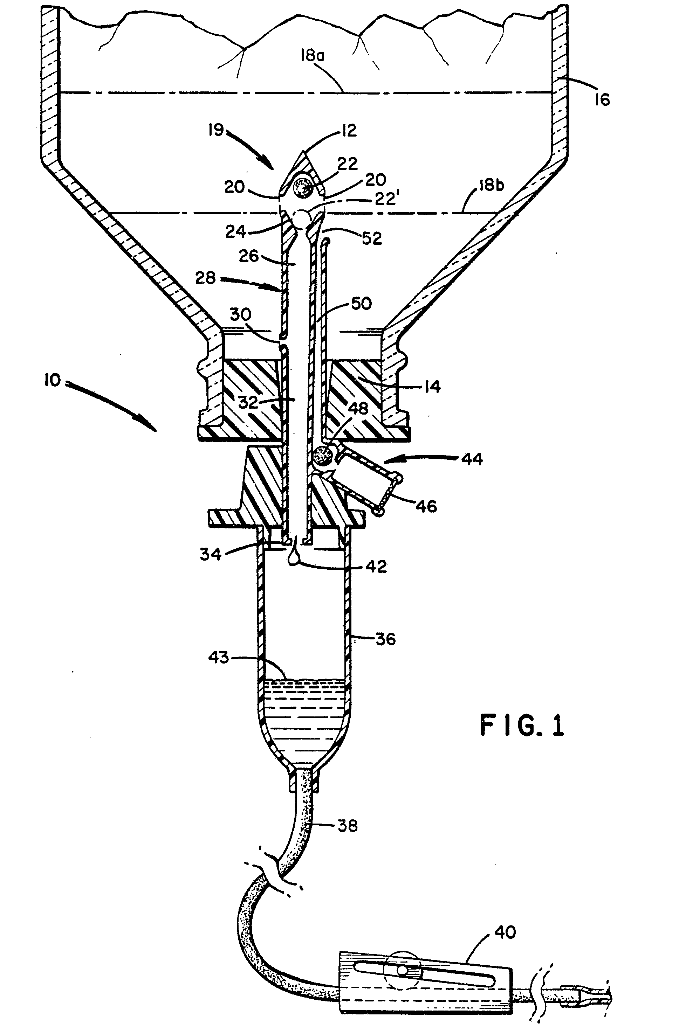

In Figure 1 the automatic flow regulator 10 of the present invention is shown having an integrally formed piercer and having the flow regulating means located entirely within the piercer and thus within an IV container. Figure 1 is a cross section in the plane of the drawing of a typical IV container and flow regulator so that the functional operation of the present invention may be seen more clearly and better understood. A typical IV solution container 16 is made of glass and the inventive device of the present invention may be inserted in the standard manner. The inventive flow regulator and piecer is provided with a sharp point 12 which may be easily inserted into and through the conventional stopper or diaphragm 14 of the IV container 16. When the IV solution is generally at least at a level 18a or above, the IV fluid mainly enters the flow regulator through the valving means, shown generally at 19, below the tip of the piercer 12. Specifically, fluid flows through the valve aperture 20, past valve float ball 22, down past tapered valve seat 24 and then down the primary infusion path 26 in piercer body 28. A small amount of fluid will also enter the piercer body 28 at a secondary infusion path which is provided at 30 and is positioned in the piercer body 28 so as to be just above the IV container stopper 14 when the piercer has been fully inserted into the container. The secondary infusion path is dimensioned such that it will permit only a slow flow rate. For example, approximately 25 ml per hour of IV fluid through the secondary infusion path will provide the desired function of maintaining fluid flow and preventing a clot formation in the IV catheter. While the infusion path 30 takes the form of a small pinhole, it can be considered as a tube, for its length is greater than its diameter. The dimensions of the fluid path in the secondary infusion path are selected by application of Poiseuille's formula relating flow through a tube to the pressure and viscosity of the fluid and to the radius and length of the tube:

$$F = \frac{\pi.P.R^4}{8.\eta.L} \; ; \text{ where}$$

F=flow rate through the tube,
P=hydrostatic pressure of the fluid,
$\eta$=viscosity of the fluid,
L=length of the tube, and
R=radius of the tube.

In applying this formula, for example, a secondary flow of 25 ml per hour will be achieved with an IV infusion fluid height of 95 cm at level 18b, a typical IV viscosity of 0.01 poise, a secondary path length of 2 mm, and a secondary path diameter of 0.0874 mm.

Fluid flowing through the secondary infusion path 30 will then combine with the fluid supplied through the primary infusion path 26 to flow down a combined infusion path 32 past a drop former 34 and into a drip chamber 36. The fluid is then supplied from the drip chamber 36 through IV tubing 38 past the tubing clamp 40 and then to the patient. A typical drop is shown at 42 and a fluid level in the drip chamber at any one time is represented at 43. The flow rate may be adjusted by clamping the tubing 38 with the tubing clamp 40 until the desired number of drops per minute are produced at the drop former 34. Conventionally, the orifice of drop former 34 is so sized that there are about 15 drops per milliliter of fluid. Therefore, a drop rate of 15 drops per minute gives a flow rate of 1 ml per minute or 60 ml per hour.

The diameter of the piercer body 28 is chosen so as to be compatible with existing IV container stoppers. Typically, such stoppers are adapted to accept, in one case, a 6.5 mm diameter piercer and, in another case, a 5.5 mm diameter piercer. Similarly, the height of the piercer body or length should be chosen such that when the fluid level in the IV container falls to such level, as shown generally at 18b, or just below the inlet apertures 20, a small amount of fluid will still remain in the IV container and will be about 25 to 50 ml.

In order to prevent air from entering the primary infusion path when the fluid level drops to level 18b, a float ball valve is provided. The float ball 22 seats on tapered valve set 24, and the ball 22 is chosen to have a specific gravity less that the IV fluid, so that it will float in the IV solution. In Figure 1, the ball 22 is shown floating when the fluid is at the level at 18a and in this manner the fluid will pass by the valve apertures 22 and will flow into the primary infusion path 26. When the fluid level drops to level 18b the ball, shown in dashed outline at 22', will seat on the valve seat 24, thereby preventing the entry of air into the primary infusion path 26. This is an important feature provided by the present invention, since if air were to enter the system it would interrupt the continuity of fluid infusion, especially in the IV tubing 38, and alter or even stop the IV solution flow. In addition, this air could possibly enter the patient's blood stream.

In order to prevent airlock inside the IV container when the inventive flow regulator is used with an unvented rigid IV container, an air entry system, shown generally at 44, is provided. This air entry system allows air to enter the IV container and has an air filter 46 to prevent dust and other particles or the like from contaminating the IV fluid. The air entry system also includes a ball valve 48, to prevent IV fluid from flowing out the air entry system, an air entry channel 50 located within the piercer tube 28, and an air outlet 52 at the end of channel 50.

Presently, there are commercially available vented rigid IV containers, and collapsible plastic IV containers. These IV containers generally require no venting or air entry system. Nevertheless, the inventive flow regulator with its air entry system, can be effectively used with such containers.

It is contemplated that all structural parts of the inventive flow regulator which are located above the drip chamber will be made of rigid plastic or other material which has been approved by the United States Food and Drug Administration (FDA). Additionally, the drip chamber 36 and IV tubing 38 are made of clear flexible FDA approved plastic which has a memory, i.e., will return to its original shape after any deformation. All joints in the assembly of the present invention are sealed by an FDA approved adhesive, solvent, or a suitable heat sealing method.

In operation, the embodiment of Figure 1 functions as set forth below. Initially, the stopper 14 of a fresh IV container 16 is pierced with the piercer tip 12 and piercer 28 is fully inserted in the bottle. The bottle is then inverted and suspended on a support stand (not shown). The associated IV tubing 38 is clamped by clamp 40; the drip chamber 36 is squeezed to force air out of the chamber into the IV bottle 16 and to permit fluid to flow into the drip chamber from the IV bottle. The tubing clamp 40 is released until fluid flows out of the distal end of the IV tubing, ensuring that no air is in this infusion tubing. The IV tubing clamp 40 is then closed, an IV catheter inserted into the patient, and the tubing connected to the IV catheter and finally the tubing clamp 40 is adjusted to regulate the infusion rate. The infusion rate may be monitored then by counting the rate of drop formation at the drop former 34.

When the IV container contains sufficient fluid, such as shown at level 18a, the fluid infusion is supplied mainly through apertures 20 of the valve mechanism 19 and thence primary infusion path 26; only a relatively small amount of fluid will be supplied through the secondary path 30. However, when the fluid level drops from continued infusion to level 18b, ball valve 19 is so positioned that any entry of fluid into the primary infusion path 26 ceases, and entry of air down this same path is prevented. At this time the fluid infusion rate automatically decreases drastically, since fluid is supplied to the patient only through the relatively small secondary path 30 at a slow rate, approximately 25 ml per hour, which is intended, as described above, to maintain fluid flow and avoid catheter clotting.

As mentioned above, the length of the piercer 28 is chosen such that when the piercer is fully inserted into the bottle 16 about 25 to 50 milliliters of fluid will remain in the IV container when the fluid level is as represented generally at 18b, that is, approximately the level of the valve apertures 20. Since the secondary flow rate is desired to be about 25 ml per hour, the attending medical personnel have approximately 1 to 2 hours then in which to replace the nearly empty IV container before the infusion stops and clotting of the IV catheter becomes a problem. Thus, this device extends from a few minutes to almost 1 to 2 hours the time the medical personnel have to replace the used IV container with a fresh one. Obviously, by increasing the length of the piercer 28 slightly, more fluid will remain in the IV container, i.e., when level 18b is reached and thus the duration of the extended infusion at a slow rate will be even longer.

In the embodiment of Figure 1, the primary flow rate is essentially unrestricted, and a typical IV infusion rate is set by a roller clamp 40 on the IV infusion tubing 38. However, by constricting the diameter of the primary flow path 26, regulation of the primary flow rate may be achieved. Therefore, no roller clamp will be needed and a variation in flow rate is achieved simply by adjusting the height of the IV container relative to the patient. For example, with the bottom of the IV container approximately 90 centimeters above the patient, the average fluid level is about 100 cm above the patient. Again using Poiseuille's formula, a primary fluid path of 3 cm length and 0.24 mm in diameter will restrict the primary flow rate to 100 ml per hour. This, in connection with the selected secondary flow rate of 25 ml per hour will provide a total flow rate of 125 ml per hour, a rate typical of most IV infusions in adults. Once again, variation in this rate is easily achieved by changing the height of the IV bottle relative to the patient. Since flow rate changes in direct proportion with IV infusion height relative to the patient, a 10% change in height will result in a 10% change in flow rate. Additionally, flow rate is quite constant and decreases only about 1 to 2% per hour, based solely on the fall in height of the IV fluid level due to depletion caused by the continued infusion into the patient.

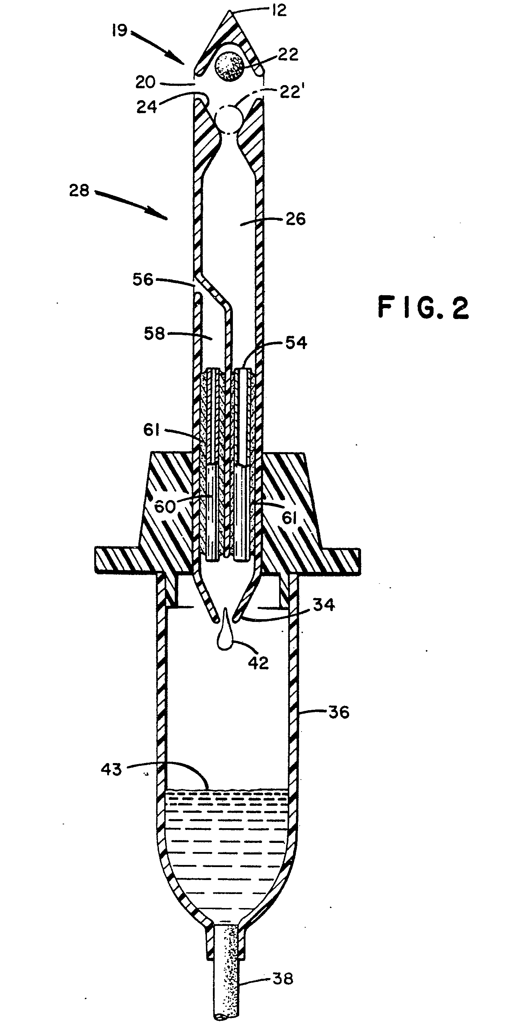

In Figure 2, another embodiment of the inventive device is shown. Because, as described above, it is not always necessary to employ an air entry system, the embodiment of Figure 2 is shown without such air entry system. Upon insertion of the embodiment of Figure 2 into a full IV container, the IV fluid will enter the primary infusion path 26 after flowing through the valve apertures 20. Fluid then flows past a flow regulating tube 54 and then through the drop former 34 into the drip chamber 36 and on into the IV tubing 38. A small amount of IV fluid will also enter into the secondary fluid inlet 56 which is so positioned as to reside just above the container stopper. Fluid then enters the secondary fluid channel 58 and flows through a flow regulating tube 60 and then into the drop former and so on. The dimensions of the flow regulating tube 54 are so chosen as to limit the total fluid flow to a rate typical of most infusion rates. The dimensions of the flow regulating tube 60 are so chosen as to limit the fluid flow to a slow, keep-open rate when the fluid level in the IV container is below the valve apertures 20, i.e., level 18b in Figure 1.

As an example, again applying the formula set forth above, a primary flow regulating tube 54 which is 2 cm long and having an internal diameter of 0.217 mm would provide a flow of 100 ml per hour with an IV fluid of the conventional viscosity and a conventional fluid head of 100 cm relative to the patient. A secondary flow regulating tube 60 then, which is also 2 cm in length but with an internal diameter of 0.155 mm, would provide a slow flow of 25 ml per hour when the IV fluid is 95 cm above the patient. Therefore, when the IV fluid is above the tip of the piercer, the fluid flow rate will be approximately 125 ml per hour and about 25 ml per hour when the IV fluid level has dropped below the apertures 20. The flow regulating tubes 54, 60 are preferrably constructed of metal or some other dimensionally stable material so that the dimensions can be accurately and reproducibly manufactured. These tubes are then positioned and sealed in place in the assembly by some suitable FDA approved adhesive 61 or the like. The other elements of the inventive flow regulator of Figure 2 may be constructed of similar materials as disclosed in relation to Figure 1.

Thus, in this embodiment, both the primary and secondary flow rates are regulated. Minor adjustments to flow may be made by adjusting the height of the IV infusion supply above the patient. In all other respects, this embodiment operates as does the apparatus disclosed in Figure 1. It should be noted, however, that, if the secondary infusion path were eliminated entirely, then the device of Figure 2 would function simply as an infusion rate flow regulator, providing accurate infusion rates which decrease only approximately 1 to 2 percent per hour based upon the depletion of the fluid level due to continuous infusion. However, the extended-infusion function will not be provided.

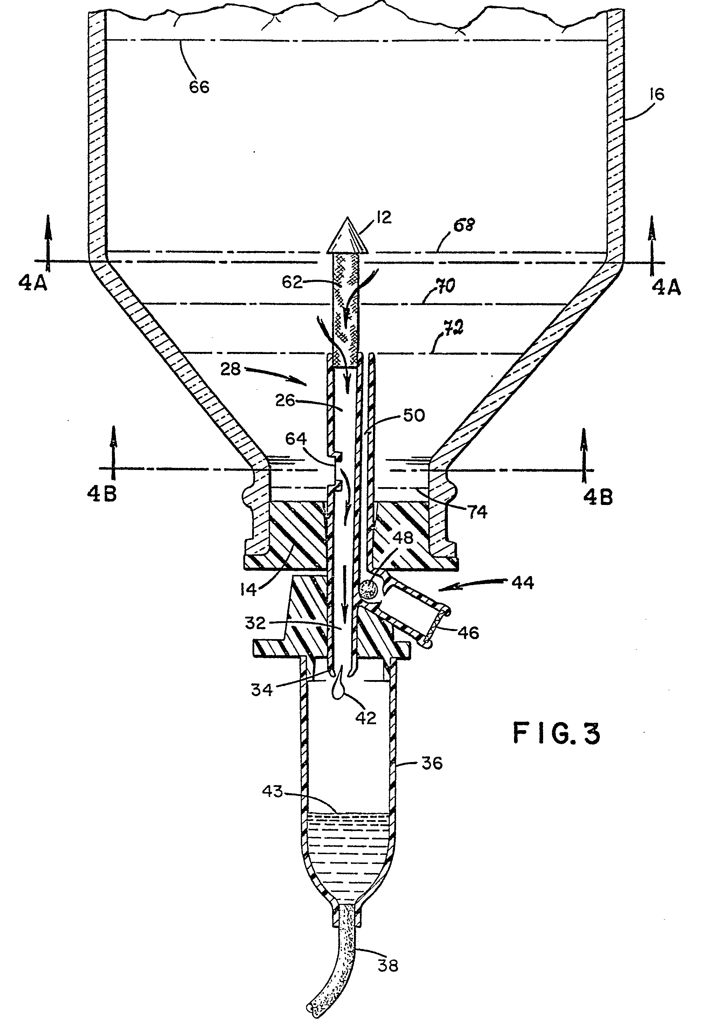

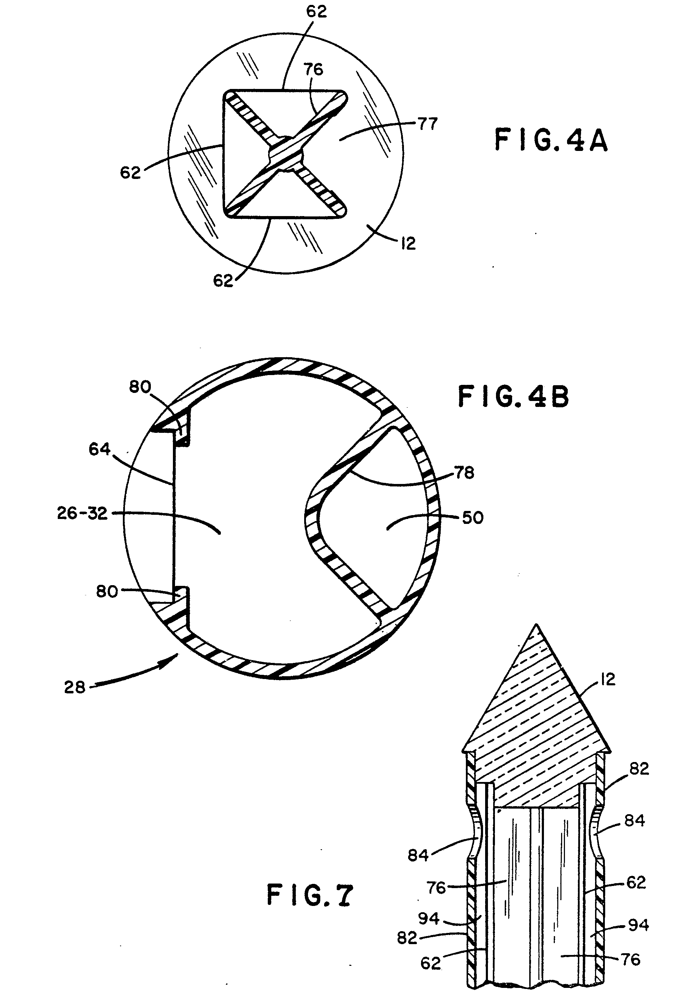

In Figure 3 another embodiment of the inventive flow regulator with integral piercer is described. Once again, the flow regulating means is located entirely within the IV piercer and hence within the IV container during operation. In the embodiment of Figure 3, a membrane filter and flow regulating means 62 is employed to allow the passage of fluid from the IV container into the primary infusion path 26. A membrane filter 64 defines the secondary fluid path. The manner in which the membranes 62 and 64 are attached to the piercer body 28 of the inventive flow regulator and piercer will be shown in more detail in Figures 4A and 4B below.

In Figure 4A, a cross section of the inventive piercer of Figure 3 is shown taken along site line 4A—4A. The primary path membrane 62 is affixed to three sides of a cross-shaped primary support structure 76 which is formed as part of the piercer tube 28. The membrane is attached to support 76 at each point of contact and especially on the ends by means of an adhesive, solvent, or heat seal approved for intravenous use by the FDA. The membrane 62 is similarly sealed at the bottom to a surface of the piercer body 28 and at the top edge to another bottom surface of the piercer tip 12, which is firmly affixed to the top of the support structure 76. In this way an air-tight seal of membrane 62 with the structural parts of the device may be achieved. The membrane 62 is slightly recessed from the outside edge of the piercer tip 12 and the piercer body 28 so as to protect the membrane as the piercer is introduced into the IV container. The open quadrant 77 of the support structure is provided to permit an outlet for the exit of air from air channel 50.

In Figure 4B, a cross section of the apparatus of Figure 3 is shown taken along site line 4B—4B wherein the secondary path filter membrane 64 is mounted on a recessed shoulder 80 of the piercer body 28. Two lines of the support structure 76 of Figure 4A continue down piercer body 28 to define a septum 78 which separates the fluid paths 26 and 32 from the air entry channel 50. It is desired that the secondary path filter membrane 64 be in a generally circular shape and it may be affixed in an air-tight manner to the recessed shoulder 80 by means of some suitably approved adhesive of the like. The shoulders 80 are provided to be recessed within the body 28 by such an amount so that the filter membrane 64 will not be abraded when the flow regulator is inserted into an IV container.

The membrane filters 62 and 64 provide filtration of the IV fluid, and membrane filters having 0.2 to 0.45 micron pores provide essentially complete filtration of bacteria and other foreign particles present in the fluid. In addition, these membrane filters are provided as being hydrophilic and, at usual IV infusion pressures and after becoming wet, they permit the passage of fluid but not air. Thus they will function as valves, closing off the infusion paths to the passage of air when fluid no longer is in contact with the filter. In addition, these membrane filters 62, 64 can be used to provide flow rate regulation, since flow through these membranes is proportional to the height of the IV infusion relative to the patient, i.e., the fluid pressure head, the pore size of the membrane filter and the effective surface area of the membrane.

Several hydrophilic membrane filters are commercially available and have been approved for use in intravenous systems by the FDA. Examples of such filters suitable for use in the present invention are those known as the Millipore 0.22 micron, type MF (GSTF), and Millipore 0.45 micron, type MF (HATF), all manufactured by the Millipore Corporation. Other membranes which may prove suitable for use in the present invention are those known as the Acropor 0.2 micron, type AN-200, and Acropor 0.45 micron, type AN-450, manufactured by the Gelman Instrument Company.

Referring once again to Figure 3, the operation of the particular embodiment will now be described.

Initially, the stopper 14 of a fresh IV container 16 is pierced with the tip 12 of the inventive device and the piercer body is fully inserted within the IV container. As described above relative to Figure 1, the bottle is then inverted and suspended on its support stand so that the stopper of the bottle is approximately 90 cm above the patient. The drip chamber 36 is then squeezed and the IV tubing clamped shut with a suitable roller clamp or the like so as to create a negative pressure within the drip chamber which serves to draw the IV fluid past the membranes 62 and 64 and into the interior of the piercer tube 28. The fluid then flows past the drop former and into the drip chamber. At this point the tubing clamp is released and the fluid flows slowly down the tubing 38. As the fluid flows further down the tubing, it will flow at a progressively higher rate since the fluid hydrostatic pressure will increase with the increasing vertical length of fluid column. When the fluid flows out of the distal end of the IV tubing 38, a clamp is closed, an IV catheter inserted in the patient, and the tubing is connected to the catheter. The clamp is opened. Then fluid begins to flow to the patient. When the inventive device is utilized with an unvented rigid container, air is fed into the IV container by way of the air entry system 44 in order to replace the fluid which has been infused and thus prevent air lock.

Since the relative area of membrane 62 is much larger than the area of 64, when the IV container fluid level is at level 68 or above, the majority of fluid will flow through membrane 62 and only a small amount of fluid will be supplied through membrane 64. As the fluid level falls to level 70 and, then to level 72, progressively less and less fluid will be passed through membrane 62 since less of the area of membrane 62 will be exposed to the fluid. However, since membrane 62 is designed to maximally pass fluid at a much greater rate than that which is normally infused, there is no significant decrease in the usual set flow rate of the infusion until the fluid almost reaches level 72. As indicated in Figure 3, at level 72 membrane 62 will be completely exposed to the air and no longer exposed to fluid. Therefore, the sole source of the infusion is through membrane 64, whose area and pore size are so selected as to limit the infusion rate to a slow rate, for example, between 15 and 35 ml per hour. The dimensions of the piercer are so chosen that when the fluid reaches level 72 only a small amount of fluid, e.g., about 25 to 50 ml of fluid, will remain in the IV container. Therefore, with a flow rate of about 25 ml per hour, the infusion is extended for approximately 1 to 2 hours longer than would normally be the case. This allows the attending medical personnel sufficient time to replace the IV container with a fresh one before the infusion stops. When a fresh IV container is added, because membranes 62 and 64 are hydrophilic, the device is still fully primed and is once again ready for use.

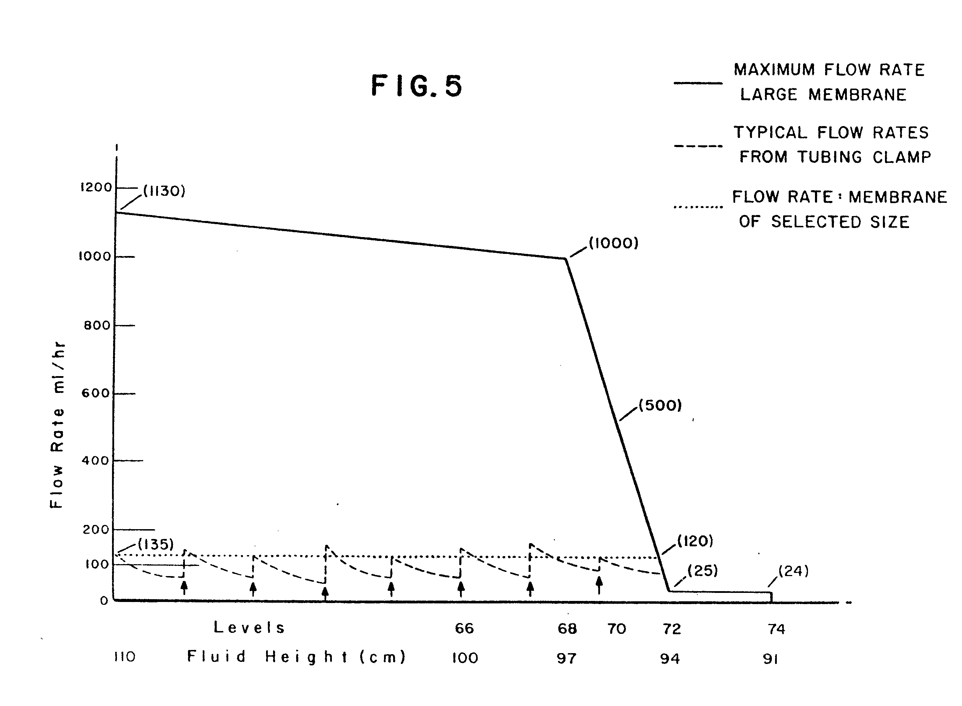

Figure 5 is a graph of calculated flow rates plotted against the fluid levels as indicated in Figure 3. Data for this graph was obtained with a Millipore 0.45 micron filter, type MF. The area of this membrane, which corresponds to membrane 62 in Figure 3, was 305 mm$^2$, and the area of membrane 64 was 8 mm$^2$. Referring then to the graph of Figure 5, the solid line represents the maximum flow rate which was achieved through the device when the IV container stopper was positioned 90 cm above the patient. When the IV container was full, the fluid level was approximately 110 cm above the patient and the maximum flow rate was then approximately 1130 ml per hour. As the fluid level fell to the level indicated at 66, which was approximately 100 cm above the patient, the maximum flow rate then decreased to approximately 1025—1030 ml per hour, a decrease which was due solely to the fall in infusion height, and thus fluid hydrostatic pressure. Upon reaching fluid level 68, membrane 62 was just barely covered with the fluid and the fluid flow rate was approximately 1000 ml per hour. At this point, there was a sharp fall off in the maximum infusion rate, since with the continued infusion both the fluid level and the effective membrane area exposed to the fluid had decreased. Upon reaching level 70, only one half of the membrane 62 was exposed to the fluid and the maximum flow rate therefore was cut essentially in half to approximately 500 ml per hour. Upon reaching level 72, the membrane 62 was longer in contact with the fluid and no fluid therefore was passed by it. At this point, the sole source of the infusion fluid was membrane 64 which passed fluid only at the rate of 25 ml per hour. Below level 72 the flow rate decreased only slightly due to the fall in infusion level to about 24 ml per hour just above level 74. When level 74 was reached the infusion stopped completely.

Since the typical IV infusion rate is only about 125 ml per hour, a rate much lower than the maximum rate as set by the membrane 62, this typical rate is then usually set by a roller clamp or the like positioned on the IV tubing. However, as may be seen from the dashed lines of Figure 5 representing the typical flow rate set by a roller clamp, this rate tends to decrease with time as much as 60% in one hour due to the above-mentioned problem of cold flow of the plastic tubing. Accordingly, this decrease requires frequent visits by the medical personnel to readjust the rate to the desired 125 ml per hour. This adjustment is indicated by the arrows just beneath the vertical portions of the dashed lines of Figure 5. In any event, as this infusion rate continues, when the membrane 62 is almost completely uncovered as the fluid reaches level 72, the infusion rate decreases to the slow rate which is set by membrane 64. This slow rate extends the IV infusion time for approximately one to two hours.

Additionally, the device in Figure 3 is able to be adapted so as to regulate both the primary and secondary flow rates and therefore avoid the use of tubing clamps. In order to provide such an adaptation, it is only necessary that membrane 62 be sized such that its effective surface area, which is presented to the fluid, is such that it limits the primary and, therefore, the total flow rate. Since flow through these membranes is proportional to the pore size, the effective surface area presented to the fluid, and the height of the infusion level relative to the patient, i.e., the fluid pressure head, accurate flow rate regulation may be easily achieved by the use of the present invention. For example, when utilizing the Millipore 0.45 micron membrane with an effective surface area of 30.5 $mm^2$, an effective flow rate of 100 ml per hour is achieved. When combining this with a secondary flow rate which has been selected at 25 ml per hour, a total flow rate of 125 ml per hour at an average fluid height of 100 cm may be provided. As indicated in Figure 5 by the dotted line, the flow will remain at approximately 125 ml per hour with only one to two percent variation per hour due to the change in infusion levels with the depletion of the fluid from the continued infusion. When membrane 62 is nearly uncovered, at reaching level 72, the secondary membrane 64 becomes the sole source of infusion, and performs as described above.

It may be appreciated from the graph of Figure 5 and the above discussion that, when compared with the erratic flow rates provided by the tubing clamp, the use of the present invention to regulate the primary flow provides a marked improvement over the previously known systems. Additionally, as mentioned above, the flow rate can also easily and accurately be adjusted by simply changing the height of the IV infusion level relative to the patient.

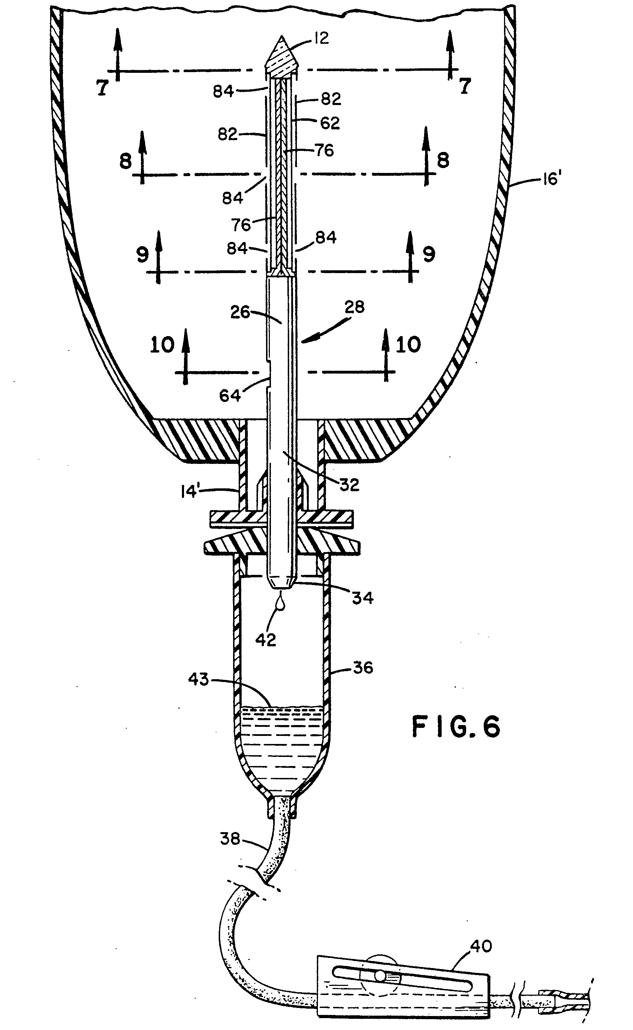

Figure 6 shows another embodiment of the present invention similar to that shown in Figure 3, and in which the inventive flow regulator and piercer provide flow regulation by apparatus which are located solely within the IV container. In this embodiment, the IV container is shown as comprising a flexible plastic container at 16'. The inventive flow regulator is provided with a fenestrated sleeve 82 which is located so as to completely surround the primary path filter membrane. This sleeve 82 is provided with several fenestrations or windows 84 which permit the fluid to come into contact with the primary membrane 62. The fenestrated sleeve 82 protects the primary path membrane 62 during the step of inserting the inventive device through the stopper or diaphragm 14' of the IV container 16'. However, the sleeve 82 is not intended to be in intimate contact with membrane 62; it is of a larger diameter, thereby permitting the fluid to come into contact with the entire surface area of the primary membrane 62. If the sleeve 82 were to be in fluid-tight contact with the membrane, then the surface area provided by the holes 84 would have to be sufficient to permit the desired primary flow rate. The embodiment of Figure 6 is not provided with an air entry system, since this system is not usually necessary with a majority of IV containers presently in use.

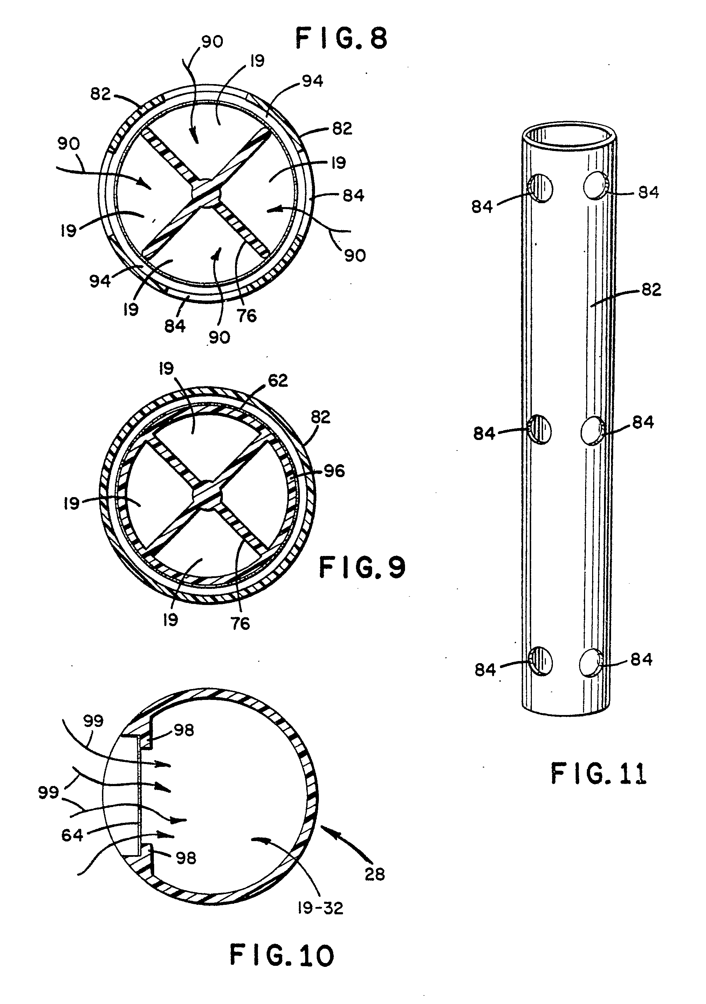

Figure 7 is a cross section of the inventive flow regulator and piercer of Figure 6 taken along site line 7—7 in the plane of the drawing. The piercer head 12 is solid and has a reduced shoulder suitable for affixing the membrane 62 and sleeve 82 thereto. The space between the sleeve 82 and the membrane 62 is shown typically at 94. Two of the several fenestrations or windows 84 are also seen in the sectional view. The piercer tip 12 is firmly affixed to membrane support 76; the membrane 62 and sleeve 82 are sealed both to air and fluid with the shoulders of the piercer tip 12 in a suitable FDA approved manner.

Figure 8 is a cross section view taken along site line 8—8 in Figure 6 and extending perpendicular to the drawing. In this figure, the inner support structure 76, which may be formed integrally with the piercer body 28, supports the primary path membrane filter 62 at the four points of contact. The membrane itself is formed at a sleeve which is surrounded by the fenestrated plastic sleeve 82. The space between the membrane 62 and the sleeve 82 is shown typically at 94. The primary fluid path 19 is then formed by the four open quadrants of the inner support structure 76 which are bounded by the primary membrane 62. Primary fluid flow through membrane 62 is indicated by the arrows 90. The membrane can be sealed to this support structure 76 but this is not necessary, since the membrane is itself formed as a sleeve and is sealed at both ends in an air and fluid tight manner.

Figure 9 is a cross section view taken along its site line 9—9 in Figure 6. The piercer body 28 is formed at this location with a reduced diameter so as to form shoulders similar to those shown in the piercer tip 12 of Figure 7. These shoulders are then used to support and to seal therewith the membrane 62 and the fenestrated sleeve 82. The membrane 62 is shown being in close contact with the shoulder 96 of the piercer body 28 and should be sealed to this shoulder in an air and fluid tight manner. As mentioned above, support structure 76 is made in one piece with the rest of the piecer body 28 or should be otherwise firmly affixed thereto. The primary fluid path 19 is now formed by the four open quadrants of the support structure 76 which are now bounded by the cross section of Figure 9 the sleeve 82 is sealed to another shoulder formed in piercer body 28 with a slightly larger diameter than shoulder 96. This may be seen more clearly in Figure 6.

Figure 10 is a cross section of the inventive device taken along site line 10—10 in Figure 6. Secondary membrane 64, which is preferably circular in shape, is intended to be sealed to both air and fluid at the circular recessed support surface 98 in the sidewall of the piercer body 28. As indicated by the arrows typically at 99 the fluid from the IV container will flow through this secondary membrane 64 and will combine with the fluid which is now flowing down the primary infusion path 19; thus, at the level of the secondary membrane 64, the primary infusion path 19 will become the combined infusion path 32.

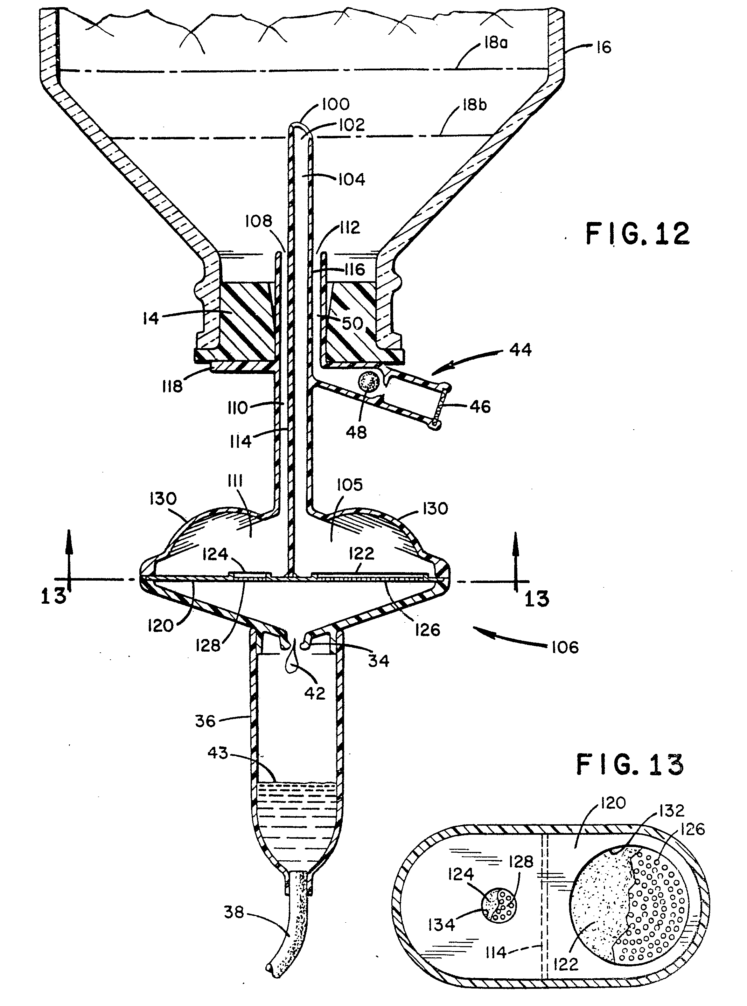

Figure 11 shows the fenestrated sleeve 82 of Figure 6 in more detail. The relative size and location of the fenestrations or windows, shown typically at 84 are also seen. Preferably there are three fenestrations per quadrant, one in the top, the middle, and the bottom of the sleeve. In this manner, the preferred embodiment employs 12 fenestrations which permit the liquid to pass through. A typical diameter of the fenestrations is approximately 2 mm. The sleeve is preferably made of a clear rigid plastic which is approved by the FDA for use in IV infusions.

In operation, the embodiment of Figure 6 functions exactly as the embodiment shown in Figure 3, with the one exception that, because the air entry system is not provided, the embodiment of Figure 6 will not operate properly in a rigid, unvented IV container. Additionally, as described in relation to the embodiment of Figure 3, the primary membrane may be chosen with dimensions so that it can provide an effective area which will limit the primary flow rate and, therefore, the total flow rate to a rate typical of the conventional IV infusion rates.

Figure 12 is another embodiment of the present invention. In this embodiment the filtering, valving, and flow regulating mechanisms are located primarily exterior to the IV container. In the embodiment of Figure 12, a piercer is provided and has a sharp tip 100 for piercing the IV container stopper or diaphragm. Arranged proximate this tip 100 is the primary flow path inlet 102, through which the IV fluid flows into the primary fluid path 104. This fluid path then continues down the piercer shaft and into a primary flow chamber 105 on the right side of the body portion of the flow regulator, which is shown generally as 106.

Located further down the piercer shaft at a position slightly above the IV container stopper innermost surface is a secondary fluit inlet 108, through which the IV fluid may enter and flow into a secondary fluid path 110. The secondary fluid path 110 then continues down the piercer shaft and into the secondary flow chamber 111 located on the left side of the body portion 106 of the flow regulator. Also formed as part of the piercer shaft is an air outlet 112, which is part of the air entry system 44, which will be required in any rigid or nonvented IV container. A septum 114 is formed by the construction of the various flow paths and serves to separate the primary fluid path 104 and chamber 105 from the secondary fluid path 110 and its respective chamber 111. Similarly, a septum 116 separates the primary fluid path 104 from the air entry system 44 to prevent communication therebetween. A stop 118 is provided towards the lower part of the shaft of the piercer and encircling the shaft of the piercer so as to allow accurate positioning of the piercer in the IV container. The stop 118 is intended to abut the outer portion of the bottle stopper or diaphragm 14. Inside the body portion of the flow regulator 106 is a membrane carrier element 120 which has mounted therein, as well as sealed thereto, a primary flow regulator and hydrophilic filter membrane 122. A secondary flow regulator and hydrophilic membrane filter 124 is also mounted in and sealed to the membrane carrier element 120. If necessary, the membrane carrier 120 can be provided with supports for the membranes such as the fenestrated membrane supports 126 and 128, which will be shown in more detail hereinbelow. Also provided in the flow regulator body 106 are two compressible domes, shown typically at 130, which are compressed after the inventive device has been inserted into an IV container in order to vent the air in the system out of the inlet 102 and 108, thereby permitting fluid to enter the primary fluid path 104 and chamber 105, as well as the secondary fluid path 110 and chamber 111. Such fluid then serves to wet the filter membrane 122 and 124 and thus to permit the flow of fluid to the patient.

Referring now to Figure 13 the membrane carrier 120 of Figure 12 is shown in more detail. As mentioned above, both the primary 122 and secondary 124 membrane filters are hydrophilic membranes, and these membranes, shown partially cut away in Figure 13, are positioned on the top surface of the membrane carrier 120 and are sealed thereto. Optional support means may be provided to support the primary and secondary membranes from beneath. Such supports may comprise fenestrated or strutted support surfaces 126 and 128 which can be incorporated as part of the membrane carrier element 120. These support surfaces are designed to support the membranes without restricting fluid flow therethrough. Therefore, the effective areas of the membranes are set by the sizes of the apertures 132 and 134 in the carrier element 120, and not by the area of the fenestrations in the support surfaces. It should be noted that, in regard to the support surfaces 126 and 128, certain of the membranes which are now commercially available are provided with a nylon mesh or other supporting backing, in which case the support structures 126 and 128 would not be necessary. The septum 114 which separates the primary flow path 104 and secondary flow path 110 continues the length of the piercer and abuts the top surface of the membrane carrier 120 in such a manner as to seal thereto. In this way, the separation between the primary and secondary fluid paths and chambers is accomplished.

Additionally, although the embodiment of Figures 12 and 13 discloses the membrane in a circular form, such form is not a necessity in order to successfully practice the present invention. The membranes may take any convenient shape, with the only constraint being a suitable surface area exposed to the fluid and a suitable membrane pore size so as to provide the desired flow rate.

The operation of the flow regulator as embodied in Figures 12 and 13 will now be described. Initially, the stopper 14 of a fresh IV container 16 is pierced with the tip 100 of the piercer and the inventive device is fully inserted into the IV container until it reaches the stop 118. The IV container is then inverted and placed on a conventional support stand. The domes 130 are alternately compressed and released, thereby forcing air out of the primary and secondary chambers 105, 111, up the primary and secondary paths 104, 110 and then into the IV container. The release of the domes draws fluid down the primary and secondary flow paths 104, 110 and into the primary and secondary chambers 105, 111, thereby wetting the filter membranes 122, 124, making the membranes permeable to the fluid. Once the membranes are wet and the air has been removed from the primary and secondary paths, the drip chamber 36 is compressed and an IV tubing clamp provided in the system is closed. This serves to draw fluid through the membranes and down into the drip chamber. At that time the tubing clamp is released and the fluid flows down the IV tubing 38; when the fluid flows out the end of the tubing device is connected to a suitable IV catheter which has already been placed in the patient. As shown above, a roller clamp located on the IV tube 38 may then be adjusted to provide the required infusion rate.

On the exhaustion of the supply of fluid in the IV container 16 the inventive piercer is removed and simply inserted into a fresh IV container. The domes 130 are once again compressed and released so as to purge any air from the secondary and primary fluid paths and to allow fluid to enter the chambers. Since the membranes will not pass air, fluid will still remain in the IV set distal to the membranes and thus no priming of this part is required. Therefore, the device is ready for use with another fresh IV container.

In operation, when the fluid supply is generally at level 18a or higher, fluid enters both the primary fluid path and the seondary fluid path and flows through the respective membranes 122, 124 into the drip chamber 36 and thence to the patient. Since membrane 122 has a pore size and surface area permitting a high flow rate when the fluid is at level 18a or above, fluid flow rate is adjusted and regulated by a tubing clamp or the like located on IV tube 38. However, once the fluid level falls to 18b, fluid no longer enters primary fluid inlet 102 and the sole source of the infusion is through the secondary inlet 108. The pore size and surface area of secondary membrane 124 are chosen so as to limit the fluid flow to a low rate, that is, a rate sufficient to prevent clotting of the intravenous catheter. Additionally, the length of the piercer is such that sufficient fluid will remain in the IV container when the fluid level is at 18b, so as to provide about one to two hours of continued infusion at the secondary flow rate. Since both membranes 122, 124 are hydrophilic and do not pass air once they have become wet, the membranes 122, 124 will perform a valving function by nature of their hydrophilic qualities and the position of the primary and secondary fluid inlets. Additionally, air will not enter the drip chamber through either membrane, thus preventing the possibility of air emboli to the patient. Also, the membranes provide a filtering function since all of the IV fluid infused into the patient must pass through either the primary or the secondary membrane before it reaches the patient.

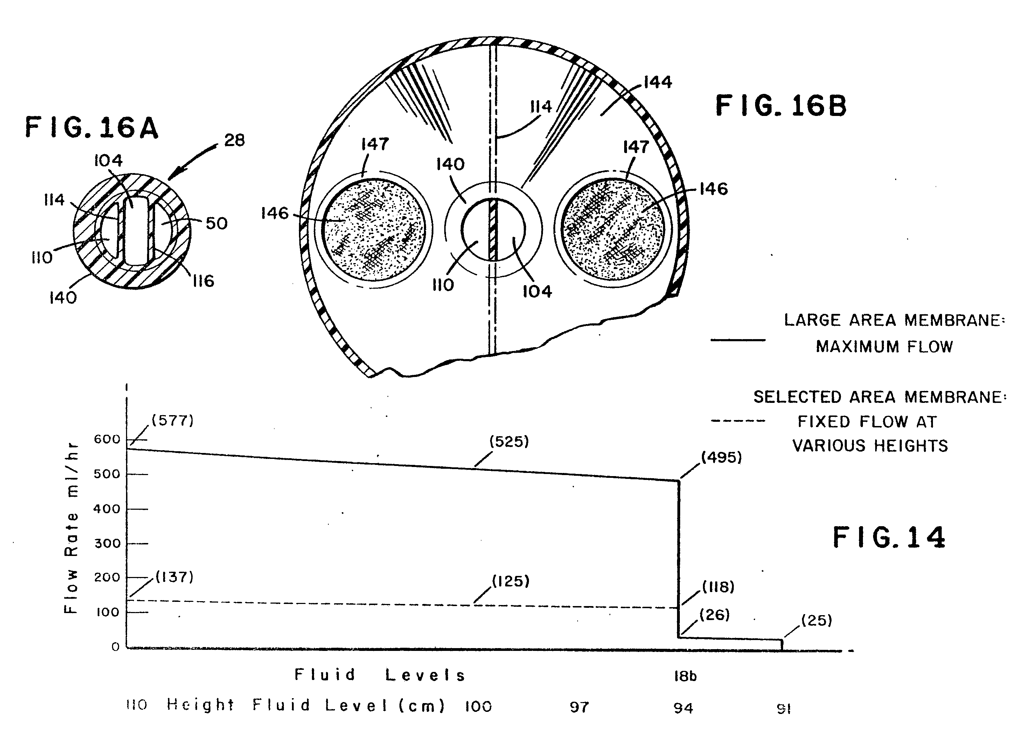

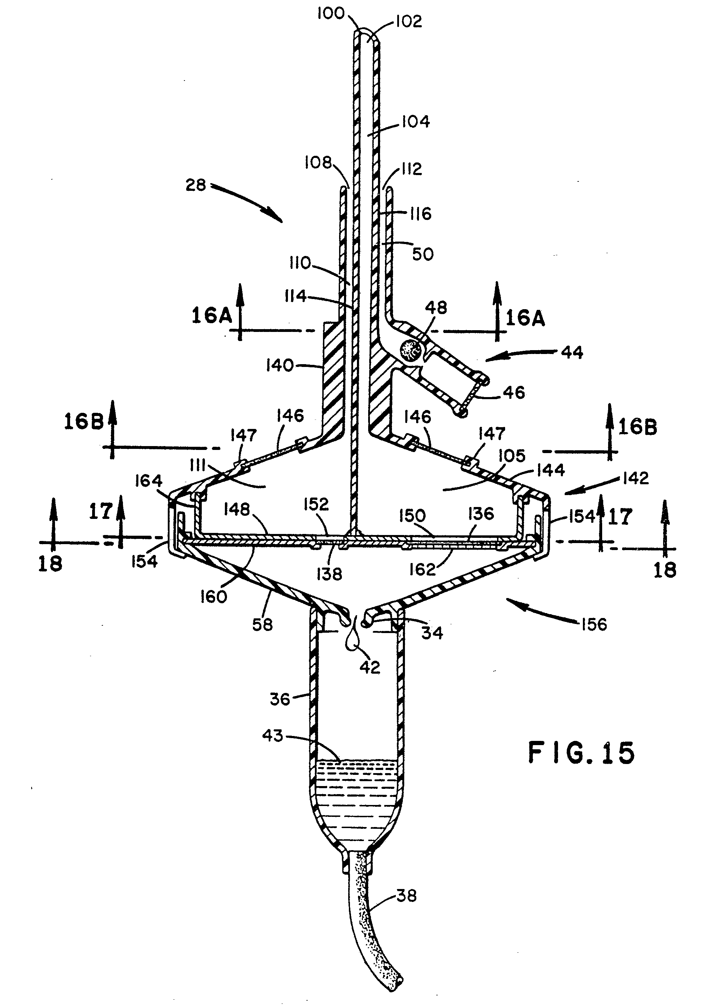

The flow pattern of the Figure 12 regulator is illustrated in Figure 14. Fluid flow rate declines abruptly when the fluid reaches the threshold level 18b. This is because the entire primary membrane will be exposed to air at one time when air enters the fluid inlet 102 and proceeds down the fluid path 104 and into primary chamber 105. For example, using a Millipore 0.22 micron filter, type MF, having a 378 $mm^2$ area for the primary membrane 122 and 19 $mm^2$ area for the secondary membrane 124, a maximum flow rate (shown in solid line) of approximately 525 ml per hour is possible when the IV fluid level is approximately 100 cm above the patient and above level 18b. This is four times greater than the usual IV flow rate. In any event, once the liquid fluid level falls to level 18b, the flow rate will decrease automatically to only about 25 ml per hour.

It may be seen that, by choosing the correct dimensions of the embodiment of Figure 12, the inventive flow regulator may easily regulate both the primary and secondary flow rates and therefore avoid the use of inaccurate tubing clamps which would be necessary in a system which could deliver 525 ml per hour. For example, using a Millipore 0.22 micron, type MF, filter membrane only 76 mm² for the primary membrane and an 19 mm² for the secondary membrane, and assuming the typical infusion height of 100 cm, a total flow rate (shown in dashed line) of only about 125 ml per hour will be provided when the fluid level is above 18b. The fluid rate will then drop to 25 ml per hour when the fluid level is below 18b. As mentioned above in relation to Figure 5, this type of flow regulation is much more accurate and stable than that which may be provided by the use of a tubing clamp or the like and, in the present inventive flow regulator, the set flow rate will decrease only approximately one to two percent per hour. Additionally, as mentioned above, the flow rate may be easily and accurately adjusted simply by raising or lowering the IV container relative to the patient and thereby changing the fluid pressure head.

Figure 15 is another embodiment of the inventive flow regulator and piercer wherein the filtration and flow regulation apparatus are located exterior to the IV container. In this embodiment, the flow regulation and filtration unit is essentially circular and is constructed of a top and bottom portion arranged so as to permit the two pieces forming the body to rotate in relation to each other. In this way, as will be described below, the flow is regulated by changing the effective surface area of the primary membrane. The piercer body 28 is provided once again with a sharp tip 100 for piercing the IV container stopper, and located adjacent this sharp tip is a primary fluid inlet 102 which permits fluid to flow into the primary fluid path 104 and subsequently into the primary chamber 105. The fluid then is passed by the primary membrane filter 136 before being introduced to the patient. Located at a point further down the piercer shaft 28 from the primary fluid inlet 102 is a secondary inlet 108 which permits fluid to enter and flow down the secondary fluid path 110 thence into the secondary chamber 111. Once again a secondary filter membrane 138 is provided in the fluid path prior to its being fed to the patient. A septum 114 separates the primary and secondary fluid paths and chambers. An air entry system 44 is provided in this embodiment which is identical to that shown in the embodiment of Figure 12. The piercer body 28 has a portion having a larger diameter 140 so as to form a shoulder which then may abut the IV bottle stopper or diaphragm. This serves to set the amount of penetration of the piercer into the IV container. As mentioned above, the body of the inventive flow regulator is provided with an upper portion shown generally at 142 which includes an outer housing surface 144 within which are fixed two hydrophobic membrane filters, shown typically at 146 and sealed in place by sealing rings 147. These hydrophobic membranes 146 will allow the venting of air out of the primary and secondary flow paths and chambers and thereby permit the fluid to enter the primary and secondary flow paths and chambers in order to wet the respective membranes. Additionally, the upper portion of the flow regulator 142 is provided with a flow regulating plate 148 which has a primary fluid aperture 150 and a secondary fluid aperture 152. Flow regulating plate 148 is sealed to the septum 114 so that primary and secondary fluid paths and chambers will remain separated at all times. A circumferential locking lip arrangement shown typically at 154 is provided in order to keep the upper body portion 142 permanently attached to the lower portion, which is shown generally at 156. The lower portion 156 has outer walls 158 which serve to collect the fluid and channel it to the drip chamber 36 which is attached to the outer walls of the lower portion. Also mounted in the lower portion 156 is a membrane carrier 160, which forms a liquid tight seal with the flow regulating plate 148. Primary membranes 136 and secondary membrane 138 are mounted in slight recesses in the membrane carrier 160 and are sealed therein. These membranes are so fixed such that a flat surface will be formed with the upper surface of the membrane carrier 160. Additionally, beneath membrane 136, membrane carrier 160 has formed as an integral part a membrane support 162 which serves to support the membrane 136 yet allow the passage of fluid therethrough. The lower portion 156 is formed with a circumferential raised lip 164 which projects up into the upper body portion 142 and is intended to coact with a corresponding lip 154 of the upper portion 142 so as to aid in forming a liquid-tight seal. If necessary, an O-ring or similar gasket might be provided at this interface so as to assure a positive liquid-tight seal. However, the sealing of the upper portion 142 with the lower portion 156 must be made in such a manner so that the two body portions may rotate relative to one another, since it is this rotation which permits the regulation of the flow rate. The operation of the rotation and the manner in which such rotation produces a variable flow rate capability is better seen in Figures 16 through 20.

Figure 16A is a cross section of the inventive flow regulator taken along site line 16A—16A of Figure 15. The main body of the piercer 28 includes the portion of increased diameter 140 which forms the shoulder for abutting against the IV container stopper. The septums 114 and 116 separate the primary path 104 from the secondary path 110 and from the air entry channel 50.

Figure 16B is a cross section taken along site line 16B—16B which is located where the piercer body 28 joins the upper portion 142 of the flow regulator. Accordingly, the enlarged diameter portion 140 of the piercer body may be seen as well as a portion of the dividing septum 114. The remainder of the septum 114 is shown in phantom since it is located beneath the top surface 144 of the upper portion. The hydrophobic membranes 146 may be sealed in place by the use of sealing rings 147. As mentioned, these hydrophobic membranes are intended to pass air but not fluid and thereby serve to vent the air from the piercer and the upper body of the device when it is first inserted into an IV container.

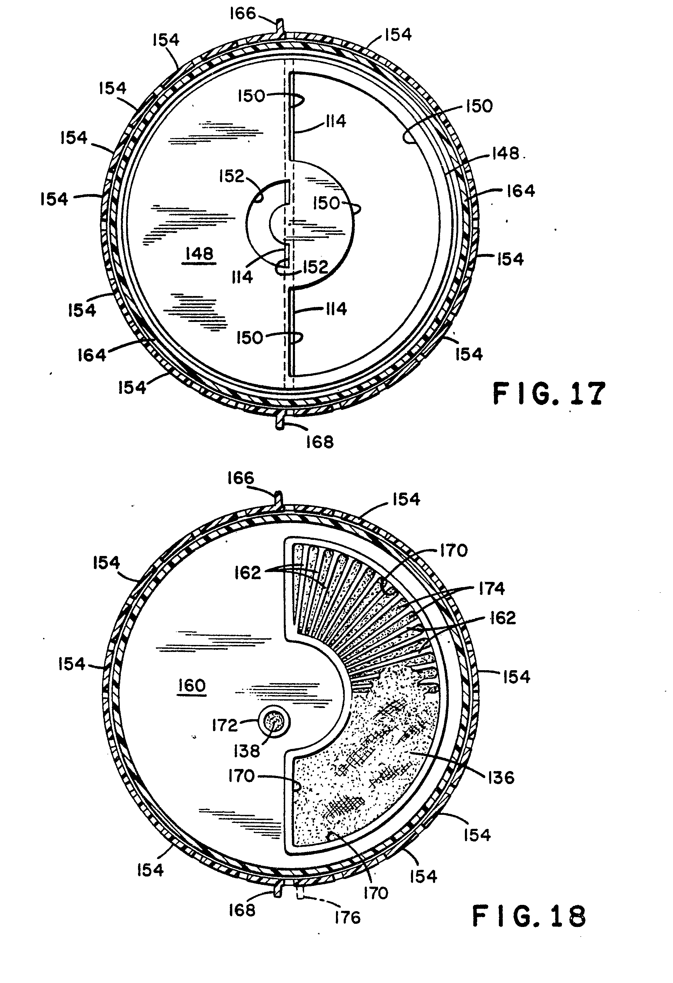

Figure 17 is a cross section of the embodiment of Figure 15 taken along site line 17—17 and which shows the flow regulating plate 148. The flow regulating plate 148 is located inside the upper portion 142 and is an integral part of the flow rate mechanism of the embodiment of Figure 15. The flow regulating plate is formed as the bottom surface of the upper body portion 142 and is intended to mate closely with the top of the membrane carrier 160 which is formed in the lower body portion 156. These two parts 148 and 160 are intended to be in as close a relationship as possible so as to form fluid-tight and air-tight seals between the solid parts of these plates. The flow regulating plate 148, as shown in Figure 17, is a solid plate except for semicircular primary flow aperture 150 and a semicircular secondary flow aperture 152. Septum 114 is seen in cross section and separates the two sides of the compression plate 148 which correspond to the primary and secondary chambers. Additionally, lip 164 of the lower body portion 156 is seen located between the outer rim of the flow regulating plate 148 and the interlocking lip structure 154 of the upper body portion 142. The interlocking lip 154 is slotted at its outer edges in several places so as to permit it to expand outwardly when it is inserted over the lower body portion 156 during the manufacturing process. Tabs 166 and 168 are affixed to lip 154 and serve to interact with a similar tab or stop on the lower body portion 156 which thereby serves to limit rotation of the upper body on the lower body to approximately one-half turn.

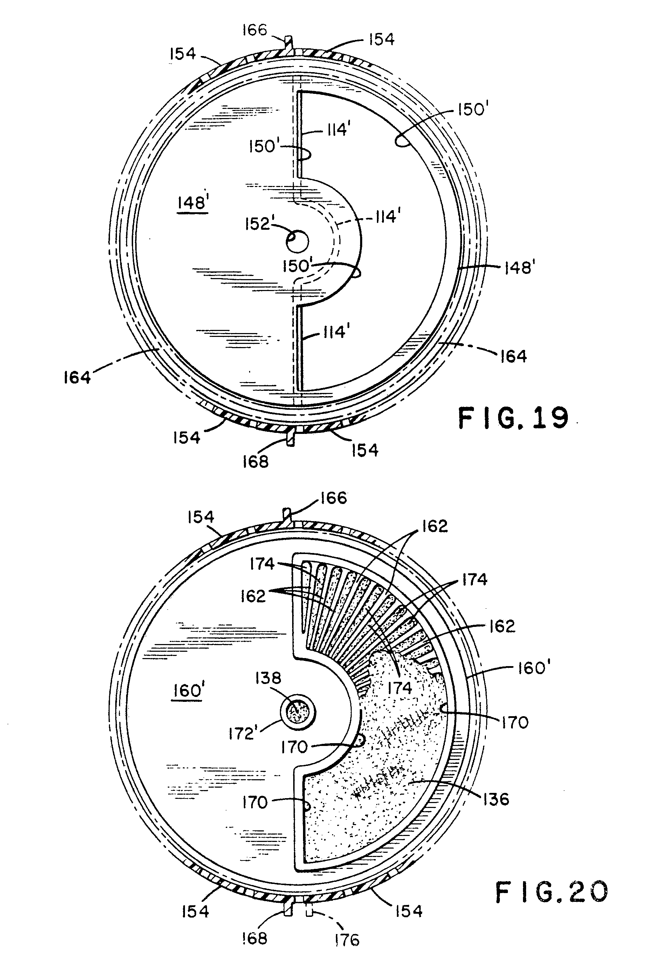

Figure 18 shows the membrane carrier 160 of Figure 15 and is a cross section taken along site line 18—18 which is just below the section referred to in Figure 17. The primary and secondary membranes 136 and 138 are shown located in their respective positions and having the majority thereof cut away for clarity of this figure. The primary membrane 136 is semicircular in shape and fits into a shallow recess 170 which is in the shape of the primary membrane. Additionally, support struts 162 project under the primary membrane 136 so as to lend it support and to keep it from deforming or sagging. The fluid flows pass the primary membrane and through the openings 174 which are formed between the adjacent struts 162. The primary membrane 136 may be sealed to the primary membrane support rim 170 and to the membrane support struts 162 by a suitable FDA approved adhesive solvent, or heat seal. The primary membrane 136 is recessed only enough so that the upper surface is flush with the topmost surface of the membrane carrier 160. Similarly, secondary membrane 138 is recessed in a flush relationship and sealed to the support rim 172. A tab or stop 176 projects from the sidewall 158 of the lower body portion 156. This stop cooperates with the stops 166 and 168, shown in Figure 16, to limit the direction and the amount of rotation of the device.

In operation, the flow regulating plate 148 is rotated on the membrane carrier 160 to regulate the flow rate through the primary fluid membrane. This rotation of the upper body and hence the flow regulating flow plate 148 upon the lower body portion and the membrane carrier 160 serves to move the primary flow aperture 150 such that it is no longer directly in alignment with the primary membrane 136. Instead, part of membrane 156 will be covered tightly by the solid portion of the flow regulating plate 148 thereby affecting the effective surface area of the primary membrane which is exposed to the fluid. Since flow rate is proportional to the effective area of the membrane filter, the flow rate is thereby accurately regulated by this rotation. In the embodiment described, complete rotation of 180° of the upper portion relative to the lower portion of the flow regulator will completely shut off the flow since, upon complete rotation, both the primary and the secondary membranes will be completely covered by solid portions of the flow regulating plate 148.

In the embodiment of Figure 18, the membranes were recessed to provide a smooth upper surface of the membrane carrier 160. An alternate embodiment is to use one large membrane which would cover and be sealed to the entire upper surface of membrane carrier 160. In this manner, fluid could flow only through the membrane where there existed openings beneath it. Thus the devices would be functionally equal. It should be noted that, in covering the entire surface with the membrane, ease of manufacture is provided as well as the additional feature that the membrane itself could act as a gasket to effect a fluid-tight seal between the movable plates 148 and 160.

Figure 19 is another embodiment of the flow regulating plate of Figure 17. In this embodiment the primary flow of aperture 150' is similar to that shown in Figure 17. However, the secondary flow path aperture 152' is now located in the center of the flow regulating plate 148'. Because of this relocation of the secondary flow path aperture 152', the septum 114' must be relocated in its relation to the plate surface. Accordingly, septum 114' deviates around the central aperture 152', so as to provide a continuous separation of the primary and secondary chambers. In all other aspects, this embodiment of the flow plate 148' retains all of the structural features as discussed relative to the plate of Figure 17.

Figure 20 is the companion alternate embodiment of the membrane carrier of Figure 18. This alternate embodiment is intended to cooperate with the flow regulating plate of Figure 19. The primary membrane 136 is semicircular in shape and is affixed to a recessed support rim 170 which is provided in the membrane carrier 160'. In order to describe this embodiment, the major portion of the primary membrane 136 has been shown cut away. Again the same support struts 162 are provided in this embodiment. Ths primary membrane 136 is therefore mounted such that the fluid can then flow through the membrane and out the multiple openings 174 which are provided between adjacent support struts 162. In this embodiment, however, the secondary membrane 138 is generally circular in shape and is mounted in a recessed support rim 172' which is centrally and axially located in the membrane carrier 160'. The secondary membrane 138 has been shown partially cut away so as to better show the manner in which the membrane is assembled.

In operation, the flow regulating plate 148' of Figure 19 is mounted directly upon and in contact with the membrane carrier 160' which has the appropriate filter membranes mounted thereon. Upon rotation of plate 148' on 160', the solid portion of plate 148' will cover a part of the surface of the membrane 136 mounted on membrane carrier 160' and thereby change the effective surface area of the primary membrane, thereby acting to regulate the flow. However, rotation of the device will not affect the effective area of the secondary membrane, since it is centrally located and is in registry with the aperture in the regulating plate. Thus, flow rate through the secondary membrane will be at all times constant and uninterrupted when the portions of the flow regulator are mutually rotated. Therefore, the tabs or stops as shown in Figures 17 and 18 are not necessary in this embodiment but have been included only to indicate the relative effective surface area of the primary membrane of this embodiment.

The operation of the embodiment of Figure 15 will now be described. Initially, the piercer 28 is inserted fully into an IV container until it is limited by the stop provided by the increased diameter portion 140. In this position, secondary inlet 108 will be located just inside the IV container and just above the end of the stopper. The IV container is then inverted and supported on a conventional stand above the patient in the typical manner. Air is vented from the upper body portion 142 of the device through the hydrophobic membranes 146, and fluid flows down the primary and secondary flow paths 104, 110 into the primary and secondary chambers 105, 111 and thence through the primary and secondary flow apertures 150, 152. Prior to insertion into the container, the upper body 142 should be rotated on the lower body portion 156 so that the flow regulator is adjusted for maximum flow. The IV fluid then wets the membranes 136 and 138. The drip chamber 36 is compressed, and the IV tubing 38 is closed; thus fluid is drawn through the membranes and into the drip chamber 36. The IV tubing 38 is then released and fluid flows down the tubing.

When the fluid flows out the distal end, the device is ready for insertion into an IV catheter which has been placed in the patient. Flow rate through the device is now at a maximum, and it may be adjusted to the desired flow rate by counting the drops formed at the drop former 34 and rotating the upper body portion 142 on the lower body portion 156 until the desired rate is achieved. The present invention will maintain the flow rate which has been set with only a one to two percent decrease per hour which is due to the falling of the pressure head of the fluid due to the continued infusion. The flow rate may be readjusted at some later time to a desired rate by again rotating the upper body portion 142 relative to the lower body portion 156, or it may be adjusted by increasing the height of the IV container above the patient.

The flow will continue at approximately the chosen rate until the IV container is almost empty, at which time the fluid will no longer be above the primary inlet 102 and the primary membrane 136 will no longer be exposed to fluid. At this time, the sole source of infusion is the secondary membrane 138 whose pore size and area are so chosen to limit the infusion to a slow rate. The IV will continue for an extended period of time at this slow rate until the IV bottle is changed or until the IV bottle becomes completely empty, at which time the infusion stops. Since the primary and secondary membranes are hydrophilic and will not pass air, no air will be permitted to enter the lower body or drip chamber or IV tubing thus avoiding the possibility of air emboli. Additionally, at the time a fresh IV container is added, air is vented from the piercer and upper body automatically through the hydrophobic membranes 146; fluid enters the chambers and rewets the membranes; and the device once against starts flowing at the set, desired infusion rate. It should also be noted that the use of the primary and secondary membranes provides complete filtration of the IV fluid used in the infusion.

Typically, the primary membrane is selected so that the effective surface area and pore size provide a maximum flow rate of from 500 to 1000 ml per hour, and the secondary membrane provides a flow rate of approximately 15 to 35 ml per hour. Also, the length of the piercer, as well as the piercer of the primary and secondary inlets, is chosen so that about 25 to 50 ml remain in the IV container when the primary inlet is no longer exposed to the fluid. This provides an extended infusion of approximately one to two hours. For example, an Acropor, 0.45 micron, membrane filter from Gelman Instrument Company having an effective surface area of 396 mm$^2$ being used as the primary membrane will provide a flow rate of approximately 1000 ml per hour at an infusion height of 100 cm. Depending on the particular configuration of the embodiment, the support struts 162 decrease the effective area by about one half and therefore allow a maximum flow through this membrane of about 500 ml per hour. It should be noted that the support struts of the embodiment of Figure 15 are in fact flow limiting, since the membrane is affixed to the support struts. The fenestrated supporting structures 126, 128 of the embodiment shown in Figure 13 are not flow limiting since the membranes are not affixed to the support structures. An Acropor, 0.45 micron, membrane filter having an area of 10 mm$^2$ utilized as a secondary membrane provides a flow of about 25 ml per hour when the infusion level is approximately 91 to 95 cm above the patient. Therefore, total maximum flow through the device is approximately 525 ml per hour. By rotating the device 45°, the flow will decrease to about 375 ml per hour; 90° to about 250 ml per hour; and 135° to about 125 ml per hour. Further rotation decreases the flow further. If the embodiments of Figure 17 and 18 are used, flow may be shut off completely by rotating the device 180°. In the embodiment of Figures 19 and 20, the flow decreases to a minimum of only about 25 ml per hour, since fluid continues to flow through the secondary flow path located in the center of the device.

It should be appreciated that the present invention contemplates regulating flow from an IV container by using filters to set maximum possible flow rate. In this regard, typical infusion rates are generally in the neighborhood of between 100 and 150 ml/hr. A filter having a preselected area and pore size can effectively set maximum flow, with great accuracy, anywhere within this typical range.

It should be understood that the specific embodiments of the present invention as described above have been presented for the purposes of illustration only and without any intention of limiting the scope of the present invention. Accordingly, it is an intention that the present invention be limited not by the above, but only as defined in the appended claims.

## Claims

1. An automatic flow regulator for delivering a fluid, such as a parenteral solution, from a fluid source (16, 16') to a patient, the flow regulator comprising: a body portion having an inlet for receiving fluid from said fluid source (16, 16'), an outlet (34) for delivering fluid to said patient, and a flow path for delivering fluid from said inlet to said outlet; an elongated piercer (28) on said body portion proximate said inlet and having a primary fluid inlet (20, 84, 102) at the end remote from said body portion, characterized by the piercer having a secondary fluid inlet (30, 56, 108) positioned intermediate said primary fluid inlet and said body portion, and having at least one piercer flow path for delivering fluid entering said primary and secondary fluid inlets to said body portion inlet; whereby when said piercer is in said fluid source and is in communication with said fluid, said primary fluid inlet defines a threshold level (18b, 72) whereabove fluid flows from source to said outlet, through said primary and secondary fluid inlets, at a first flow rate; and valving means (19, 62, 122, 136) for preventing the flow of air from said primary fluid inlet to said outlet upon the level of said fluid in said source falling below said threshold level; whereby, after the level of fluid in said source falls below said threshold level, fluid flows from said source to said outlet, through only said secondary fluid inlet, at a second flow rate less than said first flow rate.

2. The flow regulator recited in claim 1, wherein said piercer (28) extends into the interior of said source (16, 16') a predetermined distance to set said threshold level (18b, 72).

3. The flow regulator recited in claim 1, and further comprising a drop forming means (34) and an airfluid interface for enabling the monitoring of the rate of fluid infusion into a patient.

4. The flow regulator recited in claim 1, and further comprising a flow regulating tube (54) in the fluid path from said primary fluid inlet (20, 84, 102) to said outlet (34) for limiting fluid flow from said primary fluid inlet to the patient.

5. The flow regulator recited in claim 1, and further comprising a flow regulating tube (60) in the fluid path from said secondary fluid inlet (30, 56, 108) to said outlet for limiting fluid flow from said secondary fluid inlet to the patient.

6. The flow regulator recited in claim 1, and further comprising a filter membrane (64, 124, 138) in the fluid path of said secondary fluid inlet (30, 56, 108) having a preselected surface area and pore size for determining said second flow rate and for blocking the passage of air from said secondary fluid inlet to said outlet (34).

7. The flow regulator recited in claim 1, and further comprising an air inlet means (44) for allowing air to enter said source as fluid leaves said source through said primary and secondary fluid inlets.

8. The flow regulator recited in claim 1, wherein said at least one piercer flow path comprises first and second piercer flow paths, communicating, respectively, with said primary (20, 84, 102) and secondary (30, 56, 108) fluid inlets; and further comprising primary (105) and secondary (111) chambers for receiving fluid from said respective first and second piercer flow paths; and primary (62, 122, 136) and secondary (64, 124, 138) membranes mounted in said respective primary and secondary chambers and adapted to determine said first and second flow rates, respectively.

9. The flow regulator recited in claim 8, wherein said primary (62, 122, 136) and secondary (64, 124, 138) membranes are hydrophilic membrane filters; and wherein the surface area and the pore size of said respective primary and secondary membranes determines said first flow rate.

10. The flow regulator recited in claim 8, wherein said secondary membrane (64, 124, 138) is a hydrophilic membrane filter, and wherein the surface area and pore size of said secondary membrane determine said second flow rate.

11. The flow regulator recited in claim 8, and further comprising regulator means (148) for affecting the effective surface area of said primary membrane (136).

12. The flow regulator recited in claim 11, wherein said regulator means comprises a flow regulating plate (148), and wherein the relative position between said flow regulating plate and said primary membrane (136) determines the effective surface area of said primary membrane and hence the primary flow rate equal to said first flow rate less said second flow rate.

13. The flow regulator recited in claim 12, wherein said flow regulating plate (148) is adapted to block all fluid flow through said primary (136) and said secondary (138) membranes.

14. An automatic flow regulator for delivering a fluid, such as a parenteral solution, from a source (16) to a patient, the flow regulator characterized by a body formed of mutually rotatable upper (142) and lower (156) portions for receiving fluid from the source and for regulating flow to the patient; a rigid, hollow piercer (28) connected to the upper portion of said body and with a pointed tip (100) for insertion into said source; an outlet means (38) connected to the lower portion of said body for delivering fluid from said body to the patient; an primary delivery means (104, 105) residing in the upper portion of said body and in said piercer, having an inlet end (102) located near the pointed tip (100) of said piercer (28) for associating with said source, and having a discharge end in the upper portion of said body; a primary filter membrane (136) located in said body between said primary delivery means and said outlet means, said primary membrane being of selected area and pore size so as to establish a first flow rate of fluid from said primary delivery means to said outlet means and for blocking the passage of air from said primary delivery means to said outlet means; a secondary delivery means (110, 111) residing in the upper portion of said body and in said piercer, having an inlet end (108) located a predetermined distance from the pointed tip (100) of said piercer (28) for associating with said source, and having a discharge end in the upper portion of said body, said predetermined distance being larger than the distance from the inlet end (102) of said primary delivery means (104, 105) to the pointed tip (100) of said piercer (28); a secondary filter membrane (138) located in said body between said secondary delivery means and said outlet means, said secondary membrane being of selected area and pore size so as to convey fluid from said secondary delivery means to said outlet means at a second flow rate less than said first flow rate and for blocking the passage of air from said secondary delivery means to said outlet means; flow regulating plate means (148) in one of the upper or lower portions of said body, having open areas and solid areas in a predetermined ratio, said open areas communicating with the discharge ends of said primary and secondary delivery means; a membrane supporting plate (160) in the other of the upper or lower portions of said body for mounting said primary and said secondary membranes; and collecting means (36) for delivering fluid flowing through said membranes to said outlet means; whereby said flow regulating plate and said membrane supporting plate cooperate during relative rotation between said upper portion and said lower portion of said body to vary the flow of said fluid by varying the effective area of said primary membrane.

15. An automatic flow regulator for use with a closed container (16) supplying parenteral solution to a patient, the flow regulator comprising: a tubular means (28) having affixed to one end thereof a pointed piercing means (12, 100); a primary aperture (20, 84, 102) in said tubular means proximate said pointed piercing means; characterized by there being a secondary aperture (30, 56, 108) in said tubular means located at a predetermined distance from said end having said piercing means affixed thereto, said predetermined distance being larger than the distance from said primary aperture (20, 84, 102) in said tubular means to said end having said piercing means affixed thereto; a hollow body communicating with said tubular means opposite said pointed end and adapted to be arranged external to said container; flow regulator means (62, 64, 122, 124, 136, 138, 148) arranged internal to said hollow body, having a first portion communicating with said primary aperture for permitting solution to pass therethrough at a first flow rate, and having a second portion communicating with said secondary aperture permitting solution to pass therethrough at a second flow rate which is less than said first flow rate.

16. The flow regulator recited in claim 15, and further comprising a first membrane filter (62, 122, 136) arranged within said first portion of said hollow body (28, 106, 142) such that said solution in said first portion passes only through said first membrane filter; and a second membrane filter (64, 124, 138) arranged within said second portion of said hollow body such that said solution in said second portion passes only through said second membrane filter.

17. The flow regulator recited in claim 16, and further comprising a first hydrophobic membrane (146) located in the external surface of said first portion of said hollow body (142) for permitting air to pass therethrough and for preventing solution from passing therethrough, and a second hydrophobic membrane (146) located in the external surface of said second portion of said hollow body (142) for permitting air to pass therethrough and for preventing solution from passing therethrough.

18. The flow regulator recited in claim 16, and further comprising first and second collapsible domes (130) positioned in a top exterior surface of said hollow body (106) and being constructed to be manually deformed so as to reduce the volume inside said hollow body.

## Patentansprüche

1. Automatischer Durchflußregler für die Abgabe einer Nährlösung oder eines anderen Fluids aus einer Fluidquelle (16, 16') an einen Patienten, mit einem Mittelstück, an dem ein Einlaß für die Entgegennahme von Fluid aus der Fluidquelle (16, 16'), ein Auslaß (34) für die Abgabe von Fluid an den Patienten und ein Fließweg vorgesehen ist, auf welchem Fluid von dem Einlaß zu dem Auslaß gefördert wird, ferner mit einem länglichen Dorn (28) an dem Mittelstück in unmittelbarer Nähe des Einlasses, an welchem Dorn sich ein Fluidhaupteinlaß (20, 84, 102) an dem von dem Mittelstück abgewandten Ende befindet, dadurch gekennzeichnet, daß der Dorn einen Fluidnebeneinlaß (30, 56, 108) aufweist, der sich zwischen dem Fluidhaupteinlaß und dem Mittelstück befindet und der Mindestens einen Dorn-Fließweg aufweist, auf welchem Fluid, das in den Fluidhaupteinlaß und den Fluidbeneneinlaß eintritt, an den Mittelstückeinlaß gefördert wird, wodurch, wenn der Dorn sich in der Fluidquelle befindet und mit dem Fluid verbunden ist, der Fluidhaupteinlaß eine Schwellenhöhe (18b, 72) definiert, oberhalb welcher das Fluid von der Quelle zu dem Auslaß durch den Fluidhaupteinlaß und den Fluidnebeneinlaß mit einer ersten Fließgeschwindigkeit fließt, und ferner eine Sperreinrichtung (19, 62, 122, 136), die es verhindert, daß ein Luftstrom von dem Fluidhaupteinlaß in den Auslaß fließt, wenn der Pegel des Fluids in der Quelle unter die genannte Schwellenhöhe fällt, so daß, nachdem der Fluidpegel in der Quelle unter die Schwellenhöhe gefallen ist, das Fluid von der Quelle zu dem Auslaß nur durch den Fluidnebeinlaß, und zwar mit einer zweiten Fließgeschwindigkeit fließt, die geringer ist als die erste Fließgeschwindigkeit.

2. Durchflußregler nach Anspruch 1, dadurch gekennzeichnet, daß der Dorn (28) in die Quelle (16, 16') ein bestimmtes Stück weit hineinragt und dadurch die Schwellenhöhe (18b, 72) vorgibt.

3. Durchflußregler nach Anspruch 1, weiter gekennzeichnet durch eine Tropfenbildungseinrichtung (34) und eine Luft/Fluid-Grenzfläche, wodurch die Infusionsrate für den Patienten überwacht wird.

4. Durchflußregler nach Anspruch 1, weiter gekennzeichnet durch ein Durchflußreglerrohr (54) in dem Fließweg von dem Fluidhaupteinlaß (20, 84, 102) zu dem Auslaß (34) zur Begrenzung des von dem Fluidhaupteinlaß zum Patienten fließenden Fluidstroms.

5. Durchflußregler nach Anspruch 1, weiter gekennzeichnet durch ein Durchflußreglerrohr (60) in dem Fließweg von dem Fluidnebeneinlaß (30, 56, 108) zu dem Auslaß zur Begrenzung des von dem Fluidnebeneinlaß zu dem Patienten fließenden Fluidstroms.

6. Durchflußregler nach Anspruch 1, weiter gekennzeichnet durch eine Filtermembran (64, 124, 138) in dem Fließweg des Fluidnebeneinlasses (30, 56, 108) mit einer zuvor festgelegten Oberflächengröße und Porenweite zur Bestimmung der zweiten Fließgeschwindigkeit und zum Sperren des Durchtritts von Luft von dem Fluidnebeneinlaß zu dem Auslaß.

7. Durchflußregler nach Anspruch 1, weiter gekennzeichnet durch eine Lufteinlaßeinrichtung (44), durch welche Luft in die Quelle eintreten kann, wenn Fluid durch den Fluidhaupteinlaß und den Fluidnebeneinlaß aus der Quelle austritt.

8. Durchflußregler nach Anspruch 1, dadurch gekennzeichnet, daß mindestens ein Dorn-Fließweg aus einem ersten und einem zweiten Dorn-Fließweg besteht, die jeweils dem Fluidhaupteinlaß (20, 84, 102) und dem Fluidnebeneinlaß (30, 56, 108) zugeordnet sind, und ferner gekennzeichnet durch eine Hauptkammer (105) und eine Nebenkammer (111) zur Aufnahme von Fluid über den zugeordneten ersten bzw. zweiten Dorn-Fließweg, sowie durch eine Hauptmembran (62, 122,136) und eine Nebenmembran (64, 124, 138) in der zugeordneten Haupt- bzw. Nebenkammer, zur Bestimmung der ersten bzw. zweiten Fließgeschwindigkeit.

9. Durchflußregler nach Anspruch 8, dadurch gekennzeichnet, daß die Hauptmembran (62, 122, 136) und die Nebenmembran (64, 124, 138) als hydrophile Membranfilter ausgebildet sind, und daß Oberflächengröße und Porenweite der Haupt- bzw Nebenmembran die erste bzw. zweite Fließgeschwindigkeit bestimmen.

10. Durchflußregler nach Anspruch 8, dadurch gekennzeichnet, daß die Nebenmembran (64, 124, 138) als hydrophiles Membranfilter ausgebildet ist, und daß Oberflächengröße und Porenweite der Nebenmembran die zweite Fließgeschwindigkeit bestimmen.

11. Durchflußregler nach Anspruch 8, ferner gekennzeichnet durch Stellmittel (148) zum Beeinflussen der wirksamen Oberflächengröße der Hauptmembran (136).

12. Durchflußregler nach Anspruch 11, dadurch gekennzeichnet, daß die Stellmittel eine Durchflußeinstellplatte (148) aufweisen und daß die Stellung von Durchflußeinstellplatte und Hauptmembran (136) zueinander die wirksame Flächengröße der Hauptmembran bestimmen und damit die Hauptdurchflußrate gleich der ersten Durchflußrate minus der zweiten Durchflußrate ist. .

13. Durchflußregler nach Anspruch 12, dadurch gekennzeichnet, daß die Durchflußeinstellplatte (148) den gesamten Fluidstrom durch die Hauptmembran (136) und die Nebenmembran (138) abzusperren vermag.

14. Automatischer Durchflußregler für die Abgabe einer Nährlösung oder eines anderen Fluids aus einer Fluidquelle (16) an einen Patienten, gekennzeichnet durch ein Mittelstück aus zwei gegeneinander verdrehbaren Teilen, einem oberen (142) und einem unteren Teil (156), welche Teile Fluid aus der Quelle aufnehmen und den Durchfluß zum Patienten regeln, durch einen starren, hohlen Dorn (28), der mit dem oberen Teil des Mittelstücks verbunden ist und ein spitzes Ende (100) zum Einführen in die Quelle aufweist, durch eine mit dem unteren Teil des Mittelstücks verbundene Auslaßeinrichtung (38) zur Abgabe von Fluid aus dem Mittelstück an den Patienten, durch eine Hauptabgabe-einrichtung (104, 105), die sich im oberen Teil des Mittelstücks und in dem Dorn befindet und mit einem Einlaßende (102) in der Nähe des spitzen Endes (100) des Dorns (28) zum Anschließen und die Quelle und mit einem Ablaufende im oberen Teil des Hauptteils versehen ist, durch eine Hauptfiltermembran (136) in dem Mittelstück zwischen der Hauptabgabeeinrichtung und der Auslaßeinrichtung, wobei die Hauptmembran eine solche Flächengröße und Porenweite hat, daß eine erste Fluid-Durchflußrate aus der Hauptabgabeeinrichtung an die Auslaßeinrichtung entsteht, und zum Sperren des Durchtritts von Luft von der Hauptabgabeeinrichtung an die Auslaßeinrichtung, durch eine Nebenabgabeeinrichtung (110, 111) im oberen Teil des Mittelstücks und in dem Dorm, mit einem Einlaßende (108) in einem vorbestimmten Abstand von dem spitzen Ende (100) des Dorns (28) zum Verbinden mit der Quelle, und mit einem Ausgabeende in dem oberen Teil des Mittelstücks, wobei der vorbestimmte Abstand größer ist als der Abstand zwischen dem Einlaßende (102) der Hauptabgabeeinrichtung (104, 105) und dem spitzen Ende (100) des Dorns (28), durch eine Nebenfiltermembran (138) in dem Mittelstück zwischen der Nebenabgabeeinrichtung und der Auslaßeinrichtung, welche Nebenmembran vorgegebene Flächengröße und Porenweite aufweist, so daß Fluid aus der Nebenabgabeeinrichtung zu der Auslaßeinrichtung mit einer zweiten Durchflußrate geleitet wird, die niedriger ist als die erste Durchflußrate und die den Durchtritt von Luft aus der Nebenabgabeeinrichtung zu der Auslaßeinrichtung sperrt, durch eine plattenförmige Durchflußeinstelleinrichtung (148) im oberen oder im unteren Teil des Mittelstücks mit einem vorbestimmten Verhältnis zwischen durchlässigem Bereich und undurchlässigem Bereich, wobei die durchlässigen Flächenbereiche in Verbindung stehen mit den Abflußenden der Haupt- und der Nebenabgabeeinrichtung, durch einen Membranhalter (160) in dem noch ungenutzten oberen oder unteren Teil des Mittelstücks zum Haltern der Haupt- und der Nebenmembran, und durch eine Sammeleinrichtung (36) zur Abgabe von Fluid, das durch die Membranen zu der Auslaßeinrichtung fließt, wodurch die Durchflußeinstellplatte und die Membranhalterplatte bei der Relativdrehung zwischen dem oberen und dem unteren Teil des Mittelstücks zusammenwirken, um die Fluidströmung durch Veränderung der wirksamen Fläche der Hauptmembran zu verändern.

15. Automatischer Durchflußregler zur Verwendung an einem geschlossenen Behälter (16) aus dem Nährlösung an einen Patienten ausgegeben wird, mit einem Rohrteil (28), an dessen einem Ende eine zugespitzte Einstecheinrichtung (12, 100) vorgesehen ist, und mit einer Hauptöffnung (20, 84, 102) in dem Rohrteil in unmittelbarer Nähe der zugespitzten Einstecheinrichtung, dadurch gekennzeichnet, daß in dem Rohrteil eine Nebenöffnung (30, 56, 108) in einem Vorgegebenen Abstand von dem Ende mit der Daran angebrachten Einstecheinrichtung vorgesehen ist, wobei dieser vorgegebene Abstand größer ist als der Abstand zwischen der Hauptöffnung (20, 84, 102) in dem Rohrteil und dem Ende mit der daran befestigten Einstecheinrichtung, daß ein Hohlkörper mit dem Rohrteil an dem von dem zugespitzten Ende abgewandten Ende verbunden ist und außerhalb des Behälters angeordnet sein kann, daß eine Durchflußregeleinrichtung (62, 64, 122, 124, 136, 138, 148) innerhalb des Hohlkörpers vorgesehen ist und mit einem ersten Teil an die Hauptöffnung angeschlossen ist, damit Lösung mit einer ersten Durchflußrate passieren kann, und mit einem zweiten Teil an die Nebenöffnung angeschlossen ist, damit Lösung mit einer zweiten Durchflußrate passieren kann, die kleiner ist als die erste Durchflußrate.

16. Durchflußregler nach Anspruch 15, weiter gekennzeichnet durch ein erstes Membranfilter (62, 122, 136) innerhalb des ersten Teils des Hohlkörpers (28, 106, 142), so daß die Lösung in dem ersten Teil ausschließlich das erste Membranfilter durchläuft, und durch ein zweites Membranfilter (64, 124, 138) innerhalb des zweiten Teils des Hohlkörpers, so daß die Lösung in dem zweiten Teil ausschließlich das zweite Membranfilter durchläuft.

17. Durchflußregler nach Anspruch 16, weiter gekennzeichnet durch eine erste hydrophobe Membran (146) in der Außenfläche des ersten Teils des Hohlkörpers (142), so daß Luft hindurchtreten und Lösung nicht hindurchtreten kann, und durch eine zweite hydrophobe Membran (146) in der Außenseite des zweiten Teils des Hohlkörpers (142), so daß Luft hindurchtreten und Lösung nicht hindurchtreten kann.

18. Durchflußregler nach Anspruch 16, weiter gekennzeichnet durch eine in einer oberen Außenseite des Hohlkörpers (106) befindliche erste und zweite zusammenlegbare Haube (130), die so ausgebildet sind, daß sie von Hand verformt werden können, um das Volumen innerhalb des Hohlkörpers zu verkleinern.

**Revendications**

1. Régulateur de débit automatique pour acheminer un fluide, tel qu'une solution parentérale, d'une source de fluide (16, 16') vers un patient, le régulateur de débit comprenant: une partie consistant en un corps ayant une admission pour recevoir le fluide à partir de ladite source de fluide (16, 16'), d'un orifice (34) pour acheminer le fluide audit patient, et un conduit pour acheminer le fluide de ladite admission audit orifice de sortie; un perçoir allongé (28) sur ladite partie du corps à proximité de ladite admission et ayant une admission de fluide primaire (20, 84, 102) dans la partie distale de ladite partie du corps, caractérisé en ce que le perçoir présente une admission de fluide secondaire (30, 56, 108) placée entre ladite admission de fluide primaire et ladite partie du corps, et au moins un conduit de perçoir pour acheminer le fluide entrant par lesdites admissions de fluide primaire et secondaire à ladite admission se trouvant dans ledit corps; lorsque ledit perçoir est dans ladite source de fluide et est en communication avec ledit fluide, ladit admission de fluide primaire établit un niveau de seuil (18b, 72) au-dessus duquel le fluide coule de ladite source vers ledit orifice de sortie à travers lesdites admissions de fluide primaire et secondaire, et ce avec un débit de premier degré; et des valves (19, 62, 122, 136) empêchant que l'air s'écoule à partir de ladite admission de fluide primaire vers ledit orifice de sortie et que le niveau du fluide dans ladite source tombe au-dessous du niveau-seuil; après que le niveau du fluide dans ladite source tombe en dessous dudit niveau de seuil, le fluide s'écoule de ladite source vers ledit orifice de sortie uniquement à travers ladite admission de fluide secondaire, à un débit d'un moindre degré que le premier.

2. Régulateur de débit selon la revendication 1, caractérisé en ce que ledit perçoir (28) avance jusqu'à l'intérieur de ladite source (16, 16') à une distance prédéterminée pour établir ledit niveau de seuil (18b, 72).

3. Régulateur de débit selon la revendication 1, caractérisé en outre par un dispositif formant des gouttes (34) et un interface air-fluide pour permettre la surveillance du débit de l'infusion du fluide dans le patient.

4. Régulateur de débit selon la revendication 1, caractérisé en outre par un tuyau régulateur de débit (54) dans le conduit de fluide menant de ladite admission de fluide primaire (20, 84, 102) audit orifice de sortie (34) pour limiter de débit du fluide de ladite admission de fluide primaire vers le patient.

5. Régulateur de débit selon la revendication 1, caractérisé en outre par un tuyau régulateur de débit (60) dans le conduit du fluide menant de ladite admission de fluide secondaire (30, 56, 108) vers ledit orifice de sortie pour limiter le débit du fluide de ladite admission de fluide secondaire vers le patient.

6. Régulateur de débit selon la revendication 1, et caractérisé en outre par une membrane filtrante (64, 124, 138) dans le conduit de fluide de ladite admission de fluide secondaire (30, 56, 108), ladite membrane filtrante ayant une superficie et une dimension de pore prédéterminée en vue d'établir ledit débit de second degré et en vue de barrer le passage de l'air à partir de ladite admission de fluide secondaire vers ledit orifice de sortie (34).

7. Régulateur de débit selon la revendication 1, et caractérisé en outre par un dispositif d'admission d'air (44) permettant à l'air d'entrer dans ladite source au fur et à mesure que le fluide s'écoule de ladite source à travers lesdites admissions de fluide primaire et secondaire.

8. Régulateur de débit selon la revendication 1, dans lequel au moins un conduit de perçoir comprend un premier et un second conduit de perçoir communiquant respectivement avec lesdites admissions de fluide primaire (20, 84, 102) et secondaire (30, 56, 108); caractérisé en outre par des chambres primaires (105) et secondaires (111) pour recevoir le fluide à partir desdits premier et second conduits de perçoir respectivement; et des membranes primaire (62, 122, 136) et secondaire (64, 124, 138) dans lesdites chambres primaire et secondaire respectivement et adaptées pour établir lesdits permier et second débits respectivement.

9. Régulateur de débit selon la revendication 8, caractérisé en ce que lesdites membranes primaires (62, 122, 136) et secondaires (64, 124, 138) sont des membranes filtrantes hydrophiles; et en ce que la superficie et la taille des pores des membranes primaire et secondaire respectivement déterminent ledit débit de premier degré.

10. Régulateur de débit selon la revendication 8, caractérisé en ce que ladite membrane secondaire (64, 124, 138) est une membrane filtrante hydrophile et en ce que la superficie et la taille des pores de ladite membrane secondaire déterminent ledit débit de second degré.

11. Régulateur de débit selon la revendication 8, caractérisé en outre par un dispositif régulateur (148) en vue d'affecter la superficie effective de ladite membrane primaire (136).

12. Régulateur de débit selon la revendication 11, caractérisé en ce que ledit dispositif régulateur comprend une plaque de régulation de débit (148), et en ce que la position relative de ladite plaque de régulation du débit par rapport à ladite membrane primaire (136) détermine la superficie effective de ladite membrane primaire, et partant, le débit primaire qui est égal audit débit de premier degré moins ledit degré de second degré.

13. Régulateur de débit selon la revendication 12, caractérisé en ce que ladite plaque de régulation du débit (148) est adaptée afin de bloquer tout passage de fluide à travers lesdites membranes primaire (136) et secondaire (138).

14. Régulateur de débit automatique pour acheminer un fluide, tel qu'une solution parentérale, à partir d'une source (16) vers un patient, le régulateur de débit étant caractérisé par un corps formé par des parties réciproquement rotatives supérieures (142) et inférieures (156) pour la réception du fluide à partir d'une source et pour la régulation du débit vers le patient; et en ce qu'un perçoir rigide et creux (28) est connecté à la partie supérieure dudit corps et à une pointe affûtée (100) pour l'insertion dans ladite source; par un orifice de sortie (38) connecté à la partie inférieure dudit corps pour acheminer le fluide à partir dudit corps vers le patient; par un dispositif distributeur primaire (104, 105) logé dans la partie supérieure dudit corps et dans ledit perçoir, ayant une extrémité d'admission (102) logée près de la pointe affûtée (110) dudit perçoir (28) établissant le lien avec ladite source et ayant une extrémité de décharge à la partie supérieure dudit corps; en ce qu'une membrane filtrante primaire (136) est logée dans ledit corps entre ledit dispositif distributeur et ledit dispositif de sortie, ladite membrane primaire étant d'une superficie et d'une taille de pores prédéterminée en vue d'établir un débit de premier degré du fluide à partir du dispositif distributeur primaire vers le dispositif de sortie et en vue de bloquer le passage de l'air à partir dudit dispositif distributeur primaire vers ledit dispositif de sortie; par un dispositif distributeur secondaire (110, 111) se trouvant dans la partie supérieure dudit corps et dans ledit perçoir et ayant une extrémité (108) se trouvant à une distance prédéterminée de la pointe affûtée (100) dudit perçoir (28) pour s'associer avec ladite source et présentant une extrémité de décharge dans la portion supérieure dudit corps, ladite distance prédéterminée étant plus importante que la distance entre l'extrémité d'entrée (102) dudit dispositif distributeur primaire (104, 105) à la pointe affûtée (100) dudit perçoir (28); par une membrane filtrante secondaire (138) se trouvant dans ledit corps entre lesdits dispositifs distributeurs et lesdits moyens de sortie, ladite membrane secondaire étant réalisée avec une surface et une grandeur de pores sélectionnées de façon à transporter du fluide desdits dispositifs distributeurs secondaires auxdits moyens de sortie avec un deuxième débit moins important que ledit premier débit et pour bloquer le passage de l'air dudit dispositif distributeur secondaire audit dispositif de sortie; par des moyens de réglage du fluide sous forme de plaquettes (148) dans une des portions supérieures et inférieures dudit corps et présentant des surfaces ouvertes et des surfaces solides dans un rapport prédéterminé, lesdites surfaces ouvertes étant en communication avec les extrémités de décharge desdits dispositifs distributeurs primaire et secondaire; une plaque (160) supportant la membrane dans l'autre des portions supérieure et inférieure dudit corps pour monter lesdites membranes primaire et secondaire et des dispositifs de saisie (36) pour amener le fluide passant par lesdites membranes audit dispositif de sortie, et par conséquent ladite plaque de régulation du flux et ladite plaque supportant la membrane coopère pendant la rotation relative entre ladite portion supérieure et ladite portion inférieure dudit corps afin de varier le flux dudit fluide en variant la surface effective de ladite membrane primaire.

15. Régulateur de débit automatique à utiliser avec un conteneur fermé (16) livrant une solution parentérale à un patient, le régulateur de débit étant caractérisé par un dispositif tubulaire (28) ayant attaché en une de ses extrémités un dispositif perçoir affûté (12, 100), une ouverture primaire (20, 84, 102) dans ledit dispositif tubulaire se trouvant à proximité dudit dispositif perçoir affûté caractérisé en ce qu'une ouverture secondaire (30, 56, 108) dans ledit dispositif tubulaire soit placée à une distance prédéterminée de ladite extrémité et comportant le dispositif perçoir y attaché, ladite distance prédéterminée étant supérieure à la distance à partir de ladite ouverture primaire (20, 84, 102) dans ledit dispositif tubulaire et ladite extrémité à laquelle ledit dispositif perçoir est attaché; et par un corps creux communiquant avec ledit dispositif tubulaire vis-à-vis de ladite extrémité affûtée et adapté à être disposé à l'extérieur dudit conteneur; par des dispositifs régulateurs de débit (62, 64, 122, 124, 136, 138, 148) disposés à l'intérieur dudit corps creux, ayant une première partie communiquant avec ladite ouverture primaire pour permettre une solution de passer à travers à un débit de premier degré et ayant une deuxième partie communiquant avec ladite ouverture secondaire permettant la solution de passer à travers à un débit de second degré qui est inférieur audit débit de premier degré.

16. Régulateur de débit selon la revendication 15, caractérisé par une première membrane filtrante (62, 122, 136) disposée à l'intérieur de ladite première partie dudit corps creux (28, 106, 142) de manière à ce que ladite solution dans ladite première partie traverse seulement ladite membrane filtrante; et une deuxième membrane filtrante (64, 124, 138) disposée à l'intérieur de ladite seconde partie dudit corps creux de manière à ce que ladite solution dans ladite seconde partie traverse seulement à travers de ladite membrane filtrante.

17. Régulateur de débit selon la revendication 16 caractérisé en outre par une première membrane hydrophobe (146) logée sur la surface extérieure de ladite première partie et dudit corps creux (142) pour permettttre le passage de l'air à travers ledit corps creux et pour empêcher le passage de la solution à travers ce dernier, et par une deuxième membrane hydrophobe (146) logée sur la surface extérieure de ladite seconde partie dudit corps creux (142) pour permettre le passage de l'air à travers ce dernier et pour empêcher le passage de la solution à travers celui-ci.

18. Régulateur de débit selon la revendication 16 caractérisé en outre par une première et une seconde coupole repliable (130) placées au sommet de la surface extérieure dudit corps creux (106) et étant construit pour être manuellement déformé de manière à réduire le volume à l'intérieur dudit corps creux.

FIG.1

FIG. 2

FIG. 3

FIG.4A

FIG.4B

FIG.7

# FIG. 5

Legend:
- ——— MAXIMUM FLOW RATE LARGE MEMBRANE
- - - - - TYPICAL FLOW RATES FROM TUBING CLAMP
- ·········· FLOW RATE : MEMBRANE OF SELECTED SIZE

Y-axis: Flow Rate ml/hr (0, 200, 400, 600, 800, 1000, 1200)

Data labels: (1130), (1000), (500), (135), (120), (25), (24)

X-axis Levels: 66, 68, 70, 72, 74
Fluid Height (cm): 110, 100, 97, 94, 91

0001114

FIG. 6

FIG. 8

FIG. 9

FIG. 10

FIG. 11

7

FIG. 12

FIG. 13

FIG. 16A

FIG. 16B

LARGE AREA MEMBRANE:
MAXIMUM FLOW

SELECTED AREA MEMBRANE:
- - - - - FIXED FLOW AT
VARIOUS HEIGHTS

FIG. 14

Flow Rate ml/hr

600 (577)
500 (525) (495)
400
300
200 (137) (125) (118)
100 (26) (25)
0

Fluid Levels

110 Height Fluid Level (cm) 100    97    94    91

0001114

0 001 114

FIG.15

FIG. 17

FIG. 18

FIG.19

FIG.20